# EUROPEAN PATENT APPLICATION

(11) **EP 1 923 406 A1**
(43) Date of publication of application: **21.05.2008**
(21) Application number: 06796354.6
(22) Date of filing: 11.08.2006
(51) Int. Cl.: C08F 2/50, A61K 6/00, C09J 4/00

(54) **RESIN COMPOSITION**

(30) Priority: 11.08.2005 JP 2005232791
(71) Applicant: Kyowa Hakko Chemical Co., Ltd., Tokyo 103-0022 (JP)
(72) Inventor: MORIYAMA, Satoshi c/0 Kyowa Hakko Chemical Co.;Ltd, Yokkaichi-shi, Mie 510-8502 (JP); HOTTA, Iwao Kyowa Hakko Chemical Co,; Ltd, Yokkaichi-shi, Mie 510-8502 (JP); TANAKA, Masato, Tokyo 194-0004 (JP); MAKIOKA, Yoshikazu, Kanagawa 242-0001 (JP)
(74) Representative: Dossmann, Gérard
(86) International application number: PCT/JP2006/315916
(87) International publication number: WO 2007/018287

(57) **Abstract**

The present invention provides a resin composition which can be cured by visible light and whose cured product is not colored or little colored, said composition comprising a phosphine oxide compound represented by the following general formula (1) (wherein R¹ represents alkyl, alkenyl, alkynyl, cycloalkyl, cycloalkenyl, substituted or unsubstituted aryl, substituted or unsubstituted heteroaryl, substituted or unsubstituted aralkyl, or substituted or unsubstituted aralkenyl; and R², R³, R⁴, R⁵, R⁶, R⁷, R⁸ and R⁹, which may be the same or different, each represent a hydrogen atom, a halogen atom, alkyl, alkenyl, substituted or unsubstituted aralkyl, or substituted or unsubstituted aralkenyl, and two adjacent groups among R², R³, R⁴, R⁵, R⁶, R⁷, R⁸ and R⁹ may form a hydrocarbon ring together with the two carbon atoms adjacent thereto) and an unsaturated compound.

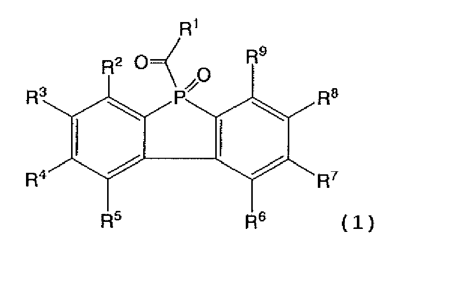

## Description

### Technical Field

The present invention relates to a resin composition comprising a phosphine oxide compound and an unsaturated compound, and the like.

### Background Art

Light curable adhesives, which are cured by light irradiation to adhere materials, are simple to use and excellent in convenience. They are preferable also from the viewpoint of environment and economy as light curing, unlike heat curing, does not need a solvent or an oven.

In general, ultraviolet light (UV) is mainly used as the irradiating light. However, in the field of precise electronic materials such as liquid crystal and organic EL (electro luminescence), use of UV curing for sealing, encapsulating and adhesion of parts sometimes causes damage and deterioration to the adjacent materials. The UV curing process also requires the step of masking. It is difficult to completely mask minute parts and failures to mask the desired spot are likely to occur, which leads to production of inferior products. Further, UV irradiation, which causes damage to irradiated materials and objects and also biological damage to workers and operators, is undesirable not only for use in the field of electronic materials, but also for biological, dental, pharmaceutical or medical use.

When a resin contains a UV absorber (UVA), a filler, a pigment, a resin, silica gel, an inorganic filler, etc. added for imparting some function to a cured product, UV is absorbed or reflected at the surface or by the absorber, resulting in insufficient curing of the inside of the product. When a resin itself absorbs UV, as in the case of a resin comprising an aromatic compound such as a benzene ring, UV is absorbed at the surface and enough amount of light does not reach the inside, which makes the curing insufficient.

Further, when an adherend is a half-transparent material (e.g., polycarbonate and polyimide) and light curing needs to be carried out though the material, UV is absorbed or reflected at the surface of the adherend and enough amount of light does not reach, which makes adhesion insufficient.

On the other hand, a visible light curable adhesive is cured by visible light, and therefore, can solve the above problems. When a visible light curable adhesive is used, materials hardly deteriorate and the operation of masking can be omitted. Unlike UV which is absorbed or reflected at the surface, visible light reaches the inside of a resin, and so the use of a visible light curable initiator enables curing of the inside of a resin comprising a filler, a resin which absorbs UV and a half-transparent adherend. Further, visible light curing has the following advantages: 1) low harmfulness to a human body when used; 2) usefulness for biological and pharmaceutical purposes; and 3) effective utilization of inexpensive light sources such as a visible light emitting diode (LED) irradiator and sunlight.

In light curing, the irradiating light (light source) is selected according to the properties of a photopolymerization initiator (absorption range, absorption intensity and active species) and the kind of a resin and the curing ability also depend upon the photopolymerization initiator. Generally, radical photopolymerization initiators of the acyl compound type are often used. The radical photopolymerization initiator cleaves a carbon-carbon bond to form a radical species, thereby starting polymerization. However, the cleavage of a carbon-carbon bond requires the photoenergy of 83.1 kcal/mol or more with a wavelength of ca. 360 nm or lower. On the other hand, among the cleavage type photopolymerization initiators, a phosphine oxide photopolymerization initiator forms a radical species by cleavage of a phosphate-carbon bond, which can be cleaved with energy a little lower than that for the cleavage of a carbon-carbon bond (wavelength: up to ca. 420 nm). Therefore, a phosphine oxide photopolymerization initiator is capable of curing with visible light (blue light: up to ca. 470 nm), and thus is one of the materials to solve the above problems. Examples of the phosphine oxide photopolymerization initiators include acylphosphine oxide (MAPO: e.g., patent document No. 1) and bisacylphosphine oxide (BAPO: e.g., patent documents Nos. 2 and 3), and they work as the initiator with blue range light and can cure resins, etc. However, in cases where a resin composition or an adhesive comprises a filler, cured product to be prepared is a thick one, an adherend is a half-transparent material, etc., their use as the photopolymerization initiator still may result in failed or insufficient curing. That is, conventional phosphine oxide photopolymerization initiators do not have practically satisfactory capability because of their insufficient ability of inner curing and visible light curing. Also known are adhesives using visible light curable photopolymerization initiators which absorb light of visible region such as camphorquinone (cQ) and titanocene (e.g., patent document No. 4), but they are not practically satisfactory because the cured product is liable to be colored or softened because of a low curing density.
Patent document No. 1:
European Patent Application No. 7508A
Patent document No. 2:
Japanese Published Unexamined Patent Application No. 101686/91
Patent document No. 3:
Japanese Published Unexamined Patent Application No. 345790/93
Patent document No. 4:
Japanese Published Unexamined Patent Application No. 313216/03

### Disclosure of the Invention

### Problems to be Solved by the Invention

An object of the present invention is to provide a resin composition which can be cured by visible light and whose cured product is not colored or little colored, and the like.

### Means for Solving the Problems

The present invention provides the following [1] to [6].
[1] A resin composition comprising a phosphine oxide compound represented by general formula (1): 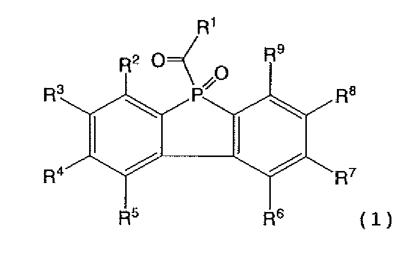 (wherein R¹ represents alkyl, alkenyl, alkynyl, cycloalkyl, cycloalkenyl, substituted or unsubstituted aryl, substituted or unsubstituted heteroaryl, substituted or unsubstituted aralkyl, or substituted or unsubstituted aralkenyl; and R², R³, R⁴, R⁵, R⁶, R⁷, R⁸ and R⁹, which may be the same or different, each represent a hydrogen atom, a halogen atom, alkyl, alkenyl, alkynyl, cycloalkenyl, alkoxy, cycloalkyloxy, alkenyloxy, cycloalkenyloxy, alkynyloxy, aryloxy, heteroaryloxy, aralkyloxy, substituted or unsubstituted aryl, substituted or unsubstituted heteroaryl, substituted or unsubstituted aralkyl, or substituted or unsubstituted aralkenyl, and two adjacent groups among R², R³, R⁴, R⁵, R⁶, R⁷, R⁸ and R⁹ may form a hydrocarbon ring together with the two carbon atoms adjacent thereto) and an unsaturated compound.
[2] The resin composition according to [1], which comprises the phosphine oxide compound wherein R¹ is substituted or unsubstituted aryl or substituted or unsubstituted heteroaryl, and R², R³, R⁴, R⁵, R⁶, R⁷, R⁸ and R⁹ each are a hydrogen atom.
[3] The resin composition according to [1], which comprises the phosphine oxide compound wherein R¹ is substituted or unsubstituted aryl, and R², R³, R⁴, R⁵, R⁶, R⁷, R⁸ and R⁹ each are a hydrogen atom.
[4] An adhesive comprising the resin composition according to [1] to [3].
[5] A cured product obtained by curing the resin composition according to [1] to [3] by light irradiation.
[6] A resin for nanoimprint comprising the resin composition according to [1] to [3].
   Hereinafter, the phosphine oxide compounds represented by general formula (1) are sometimes referred to as compounds (1).

### Effect of the Invention

The present invention can provide a resin composition which can be cured by visible light and whose cured product is not colored or little colored, and the like.

### Best Modes for carrying Out the Invention

In the definitions of the groups in the general formula, the alkyl moiety of the alkyl and the alkoxy includes straight-chain or branched alkyl groups having 1 to 20 carbon atoms, preferably 1 to 8 carbon atoms, and cycloalkyl groups having 3 to 8 carbon atoms, such as methyl, ethyl, propyl, isopropyl, butyl, isobutyl, sec-butyl, tert-butyl, pentyl, isopentyl, 2-methylbutyl, tert-pentyl, hexyl, heptyl, octyl, nonyl, decyl, dodecyl and undecyl.

The cycloalkyl moiety of the cycloalkyl and the cycloalkyloxy includes cycloalkyl groups having 3 to 8 carbon atoms such as cyclopropyl, cyclobutyl, cyclopentyl, cyclohexyl, cycloheptyl and cyclooctyl.

The alkenyl moiety of the alkenyl and the alkenyloxy includes straight-chain or branched alkenyl groups having 2 to 20 carbon atoms, preferably 2 to 10 carbon atoms, such as vinyl, allyl, propenyl, isopropenyl, butenyl, 2-methylallyl, pentenyl, hexenyl, heptenyl, octenyl, 1,3-butadienyl, 1,3-pentadienyl and 1,3,5-hexatrienyl.

The alkynyl moiety of the alkynyl and the alkynyloxy includes straight-chain or branched alkynyl groups having 2 to 20 carbon atoms, preferably 2 to 10 carbon atoms, such as ethynyl, propynyl, butynyl, pentynyl, hexynyl, 1,3-butadiynyl and 1,3,5-hexatriynyl.

The cycloalkenyl moiety of the cycloalkenyl and the cycloalkenyloxy includes cycloalkenyl groups having 3 to 20 carbon atoms, preferably 3 to 12 carbon atoms, such as cyclopropenyl, cyclobutenyl, cyclopentenyl, cyclopentadienyl, cyclohexenyl, cyclohexadienyl, cyclooctenyl, cyclododecenyl and cyclododecatrienyl.

The aryl moiety of the aryl and the aryloxy includes phenyl, naphthyl and anthryl.

The aralkyl moiety of the aralkyl and the aralkyloxy includes aralkyl groups having 7 to 30 carbon atoms, preferably 7 to 20 carbon atoms, such as benzyl, phenethyl, phenylpropyl and naphthylmethyl.

The aralkenyl includes aralkenyl groups having 8 to 30 carbon atoms, preferably 8 to 20 carbon atoms, such as styryl and cinnamyl.

The heteroaryl moiety of the heteroaryl and the heteroaryloxy includes groups in which one hydrogen atom is removed from an aromatic heterocycle. Examples of the aromatic heterocycles include 5- or 6-membered monocyclic aromatic heterocycles containing at least one atom selected from the group consisting of a nitrogen atom, an oxygen atom and a sulfur atom, and bicyclic or tricyclic fused aromatic heterocycles containing at least one atom selected from the group consisting of a nitrogen atom, an oxygen atom and a sulfur atom in which 3- to 8-membered rings are condensed, such as a pyridine ring, a pyrazine ring, a pyrimidine ring, a pyridazine ring, a quinoline ring, an isoquinoline ring, a phthalazine ring, a quinazoline ring, a quinoxaline ring, a naphthyridine ring, a cinnoline ring, a pyrrole ring, a pyrazole ring, an imidazole ring, a triazole ring, a tetrazole ring, a thiophene ring, a furan ring, a thiazole ring, an oxazole ring, an indole ring, an isoindole ring, an indazole ring, a benzimidazole ring, a benzotriazole ring, a benzothiazole ring, a benzoxazole ring, a purine ring and a carbazole ring.

The halogen atom includes a fluorine atom, a chlorine atom, a bromine atom and an iodine atom.

The hydrocarbon ring formed by two adjacent substituents among R², R³, R⁴, R⁵, R⁶, R⁷, R⁸ and R⁹ together with the two carbon atoms adjacent thereto includes unsaturated hydrocarbon rings having 5 to 10 carbon atoms, such as a cyclopentene ring, a cyclohexene ring, a cycloheptene ring, a cyclooctene ring, a benzene ring and a naphthalene ring.

The substituted aryl, the substituted heteroaryl, the substituted aralkyl and the substituted aralkenyl each have 1 to 5 substituents which are the same or different. Examples of the substituents include hydroxyl, carboxyl, a halogen atom, alkyl, alkoxy, nitro, amino which may have a substituent (examples of the substituents of the amino are those described below) and heteroaryl. The halogen atom, the alkyl moiety of the alkyl and the alkoxy and the heteroaryl have the same significances as defined above, respectively.

The substituted amino has one or two substituents which are the same or different, such as alkyl, aralkyl and aryl. The alkyl, the aralkyl and the aryl have the same significances as defined above, respectively.

The process for producing compounds (1) is described below. The following compounds represented by general formulae (2), (3) and (4) are materials for preparing compounds (1).
compounds represented by general formula (2): 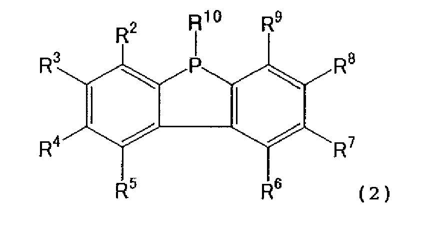 (wherein R², R³, R⁴, R⁵, R⁶, R⁷, R⁸ and R⁹ respectively have the same significances as defined above; and R¹⁰ represents alkyl, alkenyl, alkynyl, cycloalkyl, cycloalkenyl, substituted or unsubstituted aryl, substituted or unsubstituted heteroaryl, substituted or unsubstituted aralkyl, or substituted or unsubstituted aralkenyl).
compounds represented by general formula (3): 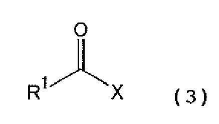 (wherein R¹ has the same significance as defined above; and X represents a halogen atom).
compounds represented by general formula (4): 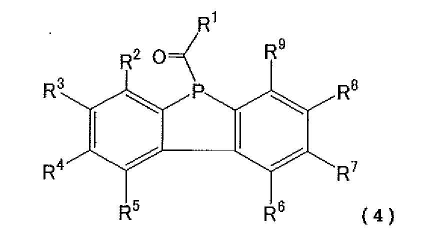 (wherein R¹, R², R³, R⁴, R⁵, R⁶, R⁷, R⁸ and R⁹ respectively have the same significances as defined above).

In the definitions of the groups in the above general formulae, the alkyl, the alkenyl, the alkynyl, the cycloalkyl, the cycloalkenyl, the substituted or unsubstituted aryl, the substituted or unsubstituted heteroaryl, the substituted or unsubstituted aralkyl, the substituted or unsubstituted aralkenyl and the halogen atom respectively have the same significances as defined above.

Hereinafter, the compounds represented by general formulae (2), (3) and (4) are sometimes referred to as compounds (2), (3) and (4), respectively.
compound (1) can be produced by oxidizing compound (4) obtained by reacting compound (2) with compound (3).
compound (2) can be produced from triarylphosphine or triarylphosphine oxide according to a known method [Hoffmann, H., chem. Ber., Vol. 95, p. 2563-2566 (1962), Bull. chem. Soc. Jpn., Vol. 64, p 3182-3184 (1991), etc.].

As the triarylphosphine, triphenylphosphine, tritolylphosphine (the substitution position may be o-, m-or p-position), tris(methoxyphenyl)phosphine (the substitution position may be o-, m- or p-position), etc. can be used. As the triarylphosphine oxide, triphenylphosphine oxide [e.g., TPP (Hokko chemical Industry co., Ltd.)], tritolylphosphine oxide [TOTP (Hokko chemical Industry co., Ltd.: the substitution position is o-position), TMTP (Hokko chemical Industry co., Ltd.: the substitution position is m-position) and TPTP (Hokko chemical Industry co., Ltd.: the substitution position is p-position)], tris(methoxyphenyl)phosphine oxide [TPAP (Hokko chemical Industry co., Ltd._{:} the substitution position is p-position)], etc. can be preferably used.
compound (4) can be synthesized by reacting compound (2) with compound (3) in an amount of preferably 0.5 to 10 equivalents, more preferably 0.8 to 2 equivalents based on compound (2) in a reaction solvent. The reaction temperature preferably starts at a low temperature (for example, -78ºc) and is gradually raised. However, the reaction is possible at temperatures below -10ºc, and after the addition of compound (3), the temperature may be raised or the reaction mixture may be heated under reflux to complete the reaction.

There is no specific restriction as to the reaction solvent so long as substrates are dissolved therein, and preferred examples include ether solvents such as tetrahydrofuran (THF), dialkyl ether, dioxane, ethylene glycol dialkyl ether and di(ethylene glycol)dialkyl ether. The amount of the solvent to be used is 1 to a large excess amount (weight ratio) based on compound (3).
compound (4) can be purified by purification methods generally employed in organic synthetic chemistry (e.g., recrystallization and chromatography) according to need.

Finally, compound (1) can be produced by oxidizing compound (4). Oxidation can be carried out by methods such as oxidation by oxygen gas, oxidation by peroxides, electric oxidation and biological oxidation. Specifically, oxidation by oxygen gas and oxidation by peroxides are preferred. When compound (4) is oxidized using oxygen gas, oxygen gas itself can be used, but preferably gases containing oxygen (e.g., air, or gas mixtures of oxygen and inert gases such as nitrogen and argon or air) are used. In oxidation by peroxides, peroxides such as an aqueous solution of hydrogen peroxide, perbenzoic acid, m-chloroperbenzoic acid, acetone oxide, tert-butylhydroperoxide and cumylhydroperoxide are used. Preferred is an aqueous solution of hydrogen peroxide.
compound (1) can be purified by purification methods generally employed in organic synthetic chemistry (e.g., recrystallization and chromatography) according to need.

The resin composition or adhesive of the present invention comprises compound (1) and an unsaturated compound. The unsaturated compounds to be used herein have one or more carbon-carbon double bonds in a molecule. They are unsaturated compounds having a low to medium or high molecular weight.

Of the unsaturated compounds having a low molecular weight, examples of the monomers having one double bond in a molecule include methyl (meth)acrylate, ethyl (meth)acrylate, butyl (meth)acrylate, methoxyethyl (meth)acrylate, methoxydiethylene (meth)acrylate, phenoxyethyl (meth)acrylate, phenoxydiethylene glycol (meth)acrylate, 2-ethylhexyl (meth)acrylate, 2-hydroxyethyl (meth)acrylate, 2-hydroxypropyl (meth)acrylate, 2-hydroxy-1-methylethyl (meth)acrylate, 4-hydroxybutyl (meth)acrylate, isobornyl (meth)acrylate, acrylonitrile, (meth)acrylamide, N-substituted (meth)acrylamide (e.g., diacetone acrylamide, N-isopropyl acrylamide, acryloylmorpholine, N,N-dimethyl acrylamide, N,N-diethyl acrylamide and N,N-dimethylpropyl acrylamide), hydroxyethylated B-naphthol (meth)acrylate, vinyl ester (e.g., vinyl acetate), vinyl ether (e.g., isobutyl vinyl ether), styrene, alkylstyrene, halostyrene, N-vinyl pyrrolidone, vinyl chloride, vinylidene chloride, 2-hydroxyethyl methacrylate (HEMA; Tokyo chemical Industry co., Ltd., or Light-Ester HO; Kyoeisha chemical co., Ltd.), 2-hydroxyethyl acrylate (HEA; Osaka Organic chemical Industry Ltd., or Light-Ester HOA; Kyoeisha chemical co., Ltd.), 4-hydroxybutyl acrylate (4-HBA; Tokyo chemical Industry co., Ltd. or Osaka Organic chemical Industry Ltd.), acryloylmorpholine (AcMO; Kohjin co., Ltd.), diacetone acrylamide (DAAM; Kyowa Hakko chemical co., Ltd.), N-isopropyl acrylamide (NIPAM; Kohjin co., Ltd.) and N,N-diethyl acrylamide (DEAA; Kohjin co., Ltd.).

Herein, the term "(meth)acrylic acid" refers to acrylic acid or methacrylic acid and the term "(meth)acrylate" refers to acrylate or methacrylate. Other derivatives are expressed in the same manner.

Of the unsaturated compounds having a low molecular weight, examples of the polyfunctional monomers having two or more double bonds in a molecule include ethylene glycol di(meth)acrylate, propylene glycol di(meth)acrylate, diethylene glycol di(meth)acrylate, triethylene glycol di(meth)acrylate, tetraethylene glycol di(meth)acrylate, 1,3-butylene glycol di(meth)acrylate, neopentyl glycol di(meth)acrylate, dipropylene glycol di(meth)acrylate, tripropylene glycol di(meth)acrylate, 1,4-butanediol di(meth)acrylate, 1,6-hexanediol di(meth)acrylate, tetramethylene glycol di(meth)acrylate, hexamethylene glycol di(meth)acrylate, bisphenol A di(meth)acrylate, 4,4'-bis(2-(meth)acryloyloxyethoxy)diphenylpropane, bisphenol A type EO (ethylene oxide)-denatured di(meth)acrylate, 9,9-bis(3-phenyl-4-(meth)acryloylpalyoxyethoxy]fluorene, tricyclodecanedimethanol di(meth)acrylate, cyclohexanedimethanol di(meth)acrylate, trimethylolpropane tri(meth)acrylate, pentaerythritol tri(meth)acrylate and tetraacrylate, pentaerythritol divinyl ether, vinyl (meth)acrylate, divinyl benzene, divinyl succinate, diallyl phthalate, triallyl phosphate, triallyl isocyanurate, tris(2-acryloylethyl) isocyanurate, divinyl ether, triethylene glycol divinyl ether, 2-hydroxy-1,3-dimethacryloxypropane and ethoxylated cyclohexane dimethanol diacrylate (NK Ester A-cHD-4E; Shin-Nakamura chemical co., Ltd.). Preferred are 1,3-butylene glycol diacrylate, 1,6-hexanediol diacrylate, neopentyl glycol diacrylate, 2-hydroxy-1,3-dimethacryloxypropane, diethylene glycol dimethacrylate, ethoxylated cyclohexane dimethanol diacrylate, tripropylene glycol diacrylate (NK Ester APG-200; Shin-Nakamura chemical co., Ltd.) and trimethylolpropane trimethacrylate.

Examples of the unsaturated compounds having a medium or high molecular weight include methoxypolyethylene glycol (meth)acrylate, phenoxypolyethylene glycol (meth)acrylate, polyethylene glycol di(meth)acrylate, polypropylene glycol di(meth)acrylate, epoxy (meth)acrylate, epoxy-denatured (meth)acrylate, polyether (meth)acrylate, urethane (meth)acrylate, polycarbonate-denatured urethane acrylate, ester (meth)acrylate, bisphenol-denatured epoxy (meth)acrylate, 2,2-bis[4-(methacryloxy·polyethoxy)phenyl]propane and unsaturated polyester resins. The preferred molecular weight of these compounds is 500 to 100,000. Preferred examples of the compounds are polycarbonate-denatured urethane acrylate (UN-9200A; Negami chemical Industrial co., Ltd.), bisphenol A type-denatured epoxy diacrylate (Ebecryl 3700 or Ebecryl 830; Daicel-UcB co., Ltd.), polypropylene glycol diacrylate #700 (NK Ester APG-700; Shin-Nakamura chemical co., Ltd.) and 2,2-bis[4-(methacryloxy·polyethoxy)phenyl]propane (NK Ester BPE-500 or NK Ester BPE-1300; Shin-Nakamura chemical co., Ltd.).

As these unsaturated compounds having a medium or high molecular weight usually have a high viscosity, they are used as the material for the resin composition or adhesive of the present invention preferably after being diluted with an unsaturated compound having a low molecular weight or a solvent.

The resin composition or adhesive of the present invention may comprise a known photoinitiator.

Examples of the known photoinitiators include hydrogen-abstraction radical initiators, benzophenone derivatives, acetophenone derivatives, benzoin ethers, benzyl ketals, monoacylphosphine oxides (e.g., 2,4,6-trimethylbenzoyl diphenylphosphine oxide; Lucirin TPO, BASF), bisacylphosphine oxides [e.g., bis(2,4,6-trimethylbenzoyl)phenylphosphine oxide; IRGAcURE 819, ciba Specialty chemical corp.], bisacylphosphine oxides, camphorquinone-amine photopolymerization initiators, peresters, titanocene photopolymerization initiators, cation photopolymerization initiators, anion photopolymerization initiators, acid generators, base generators and photopolymerization promotors. They may be used in an amount of 0.001 to 20 weight % based on compound (1). Specific examples of the hydrogen-abstraction radical initiators are 2,2-dimethoxy-1,2-diphenylethan-1-one (IRGAcURE 651; ciba Specialty chemical corp.), 1-hydroxycyclohexyl phenyl ketone (IRGAcURE 184; ciba Specialty chemical corp.), 2-hydroxy-2-methyl-1-phenylpropan-1-one (IRGAcURE 1173; ciba Specialty chemical corp.), 2-benzyl-2-dimethylamino-1-(4-morpholinophenyl)-1-butanone (IRGAcURE 369; ciba Specialty chemical corp.), 2,4-diethylthioxanthone (KAYAcURE DETX-S; Nippon Kayaku co., Ltd.), 2-cholorothioxanthone (KAYAcURE cTX; Nippon Kayaku co., Ltd.), 4'-(methylphenylthio)benzophenone (KAYAcURE BMS; Nippon Kayaku co., Ltd.) and ethylanthraquinone (KAYAcURE 2-EAQ; Nippon Kayaku co., Ltd.). Specific examples of the photopolymerization promoters are isoamyl p-dimethylaminobenzoate (KAYAcURE DMBI; Nippon Kayaku co., Ltd.) and ethyl p-dimethylaminobenzoate (KAYAcURE EPA; Nippon Kayaku co., Ltd.).

The resin composition or adhesive of the present invention may be subjected to a combination of light curing and heat curing or moisture curing. The resin composition or adhesive of the present invention may comprise, in addition to the above unsaturated compound, an epoxy resin, an oxetane resin, an urethane resin, a polyester resin, a silicone resin, a melamine resin, a fluorine resin, a polycarbonate resin, a phenol resin, a vinyl chloride resin, a vinyl acetate resin, a polyethylene resin, a polypropylene resin, a polyether resin, a polyvinyl ether resin, a polyimide resin, a polyamide resin, a polyamine resin, a polyvinyl alcohol resin, a cyanoacrylate resin, an ABS (acrylonitrile-butadiene-styrene) resin, a PET (polyethylene terephthalate) resin, a biodegradable plastic (e.g., polylactic acid), etc. The resin composition or adhesive may further comprise a heat polymerization initiator, a polymerization promoter, a moisture polymerization agent, etc. according to the resin to be contained.

The resin composition or adhesive of the present invention can comprise additives according to its purpose. An example of the additive is a polymerization inhibitor such as hydroquinone or steric hindrance phenol. The resin composition or adhesive of the present invention may comprise a copper compound, a phosphorus compound, a quaternary ammonium compound or a hydroxylamine derivative to prolong its preservation period in a dark room. The resin composition or adhesive of the present invention may further comprise paraffin or a similar wax-like substance which moves to the surface at the start of polymerization to decrease hindrances caused by oxygen during the curing. The resin composition or adhesive of the present invention may also comprise a light stabilizer. Examples of the light stabilizers are UV absorbers, UV absorbing polymers, and photodegradation inhibitors, specifically, those of the benzotriazole, benzophenone, hydroxyphenyl-s-triazine or oxalanilide type, which can be added in a small amount. More specific examples are 2-(5-methyl-2-hydroxyphenyl)benzotriazole (TINUVIN P; ciba Specialty chemical corp.), 2-(2-hydroxy-3,5-di-tert-amylphenyl)-2H-benzotriazole (TINUVIN 328; ciba Specialty chemical corp.), isooctyl 3-(3-2H-benzotriazol-2-yl)-5-tert-butyl-4-hydroxyphenylpropionate (TINUVIN 384; ciba Specialty chemical corp.), 2-[4-(2-hydroxy-3-dodecyloxypropyl)oxy}-2-hydroxyphenyl]-4,6-bis(2,4-dimethylphenyl)-1,3,5-triazine (TINUVIN 400; ciba Specialty chemical corp.), 2-[hydroxy-3,5-di(l,l-dimethylbenzyl)phenyl]-2H-benzotriazole (TINUVIN 900; ciba Specialty chemical corp.), 2-[2-hydroxy-3-dimethylbenzyl-5-(1,1,3,3-tetramethylbutyl)phenyl]-2H-benzotriazole (TINUVIN 928; ciba Specialty chemical corp.) and a hindered amine light stabilizer (HALS)-acrylic acid ester copolymer (New coat UVA or Vanaresin; Shin-Nakamura chemical co., Ltd.). The resin composition or adhesive of the present invention may also comprise a light stabilizer which does not absorb UV light (e.g., HALS light stabilizer) in order to promote light curing. Specific examples of the light stabilizers which do not absorb UV light are amines (e.g., triethanolamine, N-methyldiethanolamine, ethyl-p-dimethylaminobenzoate and Michler's ketone), a reaction product of decanedioic acid bis(2,2,6,6-tetramethyl-1-octyloxy-4-piperidinyl) ester with 1,1-dimethylethyl hydroperoxide (TINUVIN 123; ciba Specialty chemical corp.), and a mixture of bis(1,2,2,6,6-pentamethyl-4-piperidinyl) sebacate or 1-(methyl)-8-(1,2,2,6,6-pentamethyl-4-piperidinyl) sebacate (TINUVIN 292; ciba Specialty chemical corp.). Addition of an aromatic ketone of the benzophenone type to the resin composition or adhesive of the present invention can promote light curing. Addition of a photosensitive agent to the resin composition or adhesive of the present invention further promotes light curing. Examples of the photosensitive agents are aromatic carbonyl compounds (e.g., benzophenone, thioxanthone, anthraquinone and 3-acylcoumarin derivatives) and 3-(aroylmethylene)thiazolines. In addition to the above additives, the resin composition or adhesive of the present invention may also comprise, according to the purpose, a fluorescent whitening agent, a filler, a pigment, a dye, a humectant, a dispersant, an antioxidant, a lubricant, a corrosion inhibitor, an anti-alga agent, an anti-stain agent, an antistatic agent, a release agent, a flow adjusting agent, etc. The amount of the above additives in the resin composition or adhesive of the present invention is preferably 0.01 to 5 weight %.

The resin composition or adhesive of the present invention may also comprise a silane coupling agent, a hydroxy group-containing (meth)acrylate, a chelating agent, a metal trapping agent, an epoxy compound, a sulfur-containing compound, etc. to improve the cohesiveness and adhesiveness. Examples of the silane coupling agents are γ-glycidoxypropyltrimethoxysilane (KBM-403; Shin-Etsu chemical co., Ltd.), γ-glycidoxypropyl methyldiethoxysilane (KBM-402; Shin-Etsu chemical co., Ltd.), γ-glycidoxypropyltriethoxysilane (KBE-402; Shin-Etsu chemical co., Ltd.), vinyltrimethoxysilane (KBM-1003; Shin-Etsu chemical co., Ltd.), vinyltriethoxysilane (KBE-1003; Shin-Etsu chemical co., Ltd.), p-styryltrimethoxysilane (KBM-1403; Shin-Etsu chemical co., Ltd.), γ-methacryloxypropyltrimethoxysilane (KBM-503; Shin-Etsu chemical co., Ltd.), γ-methacryloxypropylmethyldimethoxysilane (KBM-502; Shin-Etsu chemical co., Ltd.), γ-methacryloxypropyltriethoxysilane (KBE-503; Shin-Etsu chemical co., Ltd.), γ-methacryloxypropylmethyldiethoxysilane (KBE-503; Shin-Etsu chemical co., Ltd.), γ-acryloxypropyltrimethoxysilane (KBM-5103; Shin-Etsu chemical co., Ltd.), γ-aminopropyltrimethoxysilane (KBM-903; Shin-Etsu chemical co., Ltd.), γ-aminopropyltriethoxysilane (KBE-903; Shin-Etsu chemical co., Ltd.), γ-mercaptopropyltrimethoxysilane (KBM-803; Shin-Etsu chemical co., Ltd.) and γ-mercaptopropylmethyldimethoxysilane (KBM-802; Shin-Etsu chemical co., Ltd.). Examples of the chelating agents are ethylenediaminetetraacetic acid (EDTA), a sodium salt of EDTA, a potassium salt of EDTA, an ammonium salt of EDTA, N,N-bis(2-hydroxyethyl)glycine, diaminopropanol tetraacetic acid and 1,6-hexamethylenediamine-N,N,N',N'-tetraacetic acid.

In the resin composition or adhesive of the present invention, compound (1) is contained preferably in an amount of 0.01 to 15 weight %, further preferably 0.2 to 5 weight % based on the total solid content.

The resin composition or adhesive of the present invention may be dissolved or dispersed in a solvent or water depending upon the structure of an unsaturated compound which constitutes the composition. Examples of the unsaturated compounds to be used here are unsaturated compounds prepared by water-solublizing or water-dispersing unsaturated compounds having a medium or high molecular weight (e.g., epoxy acrylate, polyether acrylate, urethane acrylate, ester acrylate and an unsaturated polyester resin), and water-soluble monofunctional-polyfunctional monomers (e.g., Poval). Examples of the methods for water-solublization or water-dispersion include a method which comprises introducing an acidic group (e.g., carboxylic acid and sulfonic acid) into a molecule of the above unsaturated compound and neutralizing the compound using alkali for hydrophilization, and a method which comprises introducing a polyethylene glycol unit into a molecule for hydrophilization.

Alternatively, instead of introducing a hydrophilic functional group into a molecule, known emulsifiers or surfactants may be used for water-dispersing the resin composition or adhesive of the present invention. Examples of the emulsifiers or surfactants include anionic, cationic and nonionic surfactants or high-molecular-weight emulsifiers, specifically, anionic surfactants such as higher alcohol sulfuric acid esters, alkylbenzene sulfonate, polyoxyethylene alkylsulfate and polyoxyethylene alkylphenol ether sulfate, nonionic surfactants such as polyoxyethylene alkylphenol ether, ethylene oxide-propylene oxide block polymer and sorbitan derivatives, and membrane protein solubilizers such as cHAPS [3-[(3-cholamidopropyl)dimethylaminonio]propanesulfonate; Dojindo Laboratories], cHAPSO [3-[(3-cholamidopropyl)dimethylaminonio]-2-hydroxypropanesulfonate; Dojindo Laboratories] and BIGcHAPS [N,N-bis(3-D-glucoamidopropyl)deoxycholamide; Dojindo Laboratories].

The resin composition or adhesive of the present invention may comprise, as further additives, a dispersion aid, a filler (e.g., talc, gypsum, silica, rutile, carbon black, zinc oxide or iron oxide), an extending agent, a matting agent, a defoamer, a fluorescent agent, a phosphorescent agent, a luminous agent, an electric conducting agent, metal granules (e.g., gold granules, silver granules or copper granules), a dying agent, an antibacterial agent (e.g., titanium oxide or an antibacterial organic compound), a photocatalyst, a reaction catalyst, a solid acid, an ion exchange resin, a coating, a water coating, a powder coating and other auxiliaries conventionally used in surface-coating technology.

The resin composition or adhesive of the present invention may comprise an epoxy compound, an oxetane compound, a tetrahydropyran derivative or the like which is capable of ring-opening polymerization and a cationic photopolymerization initiator or a curing agent which is capable of initiating polymerization (e.g., an amine, a carboxylic acid, an acid anhydride or a thiol compound) to reduce shrinking at the time of curing. The resin composition or adhesive of the present invention may also comprise a colorless transparent filler, a colored filler, a glossy filler or the like for reduction of shrinking. Examples of the colorless transparent fillers are silica gel, functional silica gel (functional group modified silica gel), glass (glass beads and glass pieces), titanium oxide, plastic granules (e.g., polystyrene granules, polyacryl granules, polycarbonate granules and PET granules), dental filling resin, water, aqueous solutions, saccharides, organic solvents, inorganic solids and ionic fluids. Examples of the colored fillers are pigments, dyes, opaque plastic granules, papers, pottery, latexes, emulsions, carbon black (charcoal), pebbles, sand, soil, concrete, asphalt, minerals, fertilizers, petals, seeds, pollens, soap, proteins, magnet powder, iron sand, fat, hair, skin and smoke. Examples of the glossy fillers are metal granules or metal pieces (e.g., gold, silver, copper, iron, lead, tin, aluminum, chromium, nickel, zinc, mercury, arsenic, sodium and potassium), alloys (e.g., tin plate, bronze, brass, anodized aluminum and amalgam), metal oxides (e.g., rust and verdigris), silicon wafer pieces and mirror pieces.

The resin composition or adhesive of the present invention may comprise a filler which serves as a photocatalyst such as titanium oxide or silver. In that case, a cured product obtained by curing the resin composition or adhesive of the present invention is excellent in antibacterial, antiseptic, antifouling, odor-eliminating, deodorizing and purifying properties.

The resin composition or adhesive of the present invention is cured by irradiation with sunlight, visible laser beam, UV light or the like. Preferred examples of light sources are low-pressure, medium-pressure and high-pressure mercury lamps, metal halogen lamps and laser beams whose maximum irradiation wavelength is within the range of 250 to 450 nm. When the resin composition or adhesive of the present invention comprises a photosensitizer, a long-wavelength light, for example, a laser beam up to ca. 600 nm can be used. A cured product obtained by curing the resin composition or adhesive of the present invention can be used as films, plastics, etc.

The adhesive of the present invention may be applied to adherend substrates of any material. The materials of the adherend substrates include, for example, glass, oxide film-coated glass (e.g., ITO: indium titanium oxide-coated glass), metals (e.g., aluminum, gold, silver, copper, iron, brass plate and tin plate), plastics (e.g., polycarbonate resins, acryl resins, PET resins and ABS resin plates), films (e.g., polyimide resin, vinyl chloride resin, polystyrene resin and saran resin films), papers (e.g., office papers, posters and drawing papers), building materials (e.g., slates, blocks, bricks and gypsum boards), porcelain (e.g., pottery, ceramics and tiles) and woods. The viscosity and adhesiveness can be adjusted according to the purpose of use. As the adhesive of the present invention is cured by light, materials which are light permeable or have crevices are preferred, and it is more preferred that both or either of the adherend substrates is transparent. When the adhesive layer is thick, it can be cured by light irradiation through a crevice.

The resin composition of the present invention is useful as a printing ink, varnish, a white paint for woods or metals, a coating composition for papers, woods, metals or plastics, a pigment-colored paint, an architectural finishing paint, a paint for road signs, a paint containing a visible light-curable UV absorber, a transparent or colored water-dispersed paint, a material for construction and architecture, an architectural repairing material, a road repairing material, a road line paint, an adhesive for earth and rocks, a hardening agent for earth and sand, a heavy duty anti-corrosion coating, a paint for cold districts, a fluorescent paint, a luminous paint, a lining material, a sealing material, a sealing agent, a plastic material, a fluorescent plastic, a luminous plastic, a material for photofabrication, a material for micro parts, a material for molecular devices, a material for light-curable packages and containers, a printing ink, a material for manufacturing printing plates, a material for manufacturing masks for screen printing, printed matters, a material for printing labels, a material for printing packages, a material for printing beverage packs, a material for printing business forms, a material for printing cards, a material for a photo-reproduction process, a material for photo development, a picture-recording process, a material for optical recording, a dental filling material, a dental adhesive, a medical adhesive, a material for organ reproduction, a material for plaster casts, nail art goods, a material for designing, a material for designing, a material for making a model of a small apparatus, a material for making a simulation model, a photomarking agent, an adhesive, a biological material, a material for packing animal- and plant-derived crude drugs, an etching agent for print electronic circuits, a resist material, a material for FPD (flat panel display), a material for a color filter, a film material, an electrically conductive film, an adhesive for liquid crystals, an adhesive for ITO plates, an electrically conductive adhesive, a reflection preventing film, a surface-coating material, a liquid crystal sealing agent, a prism sheet, a solder stop mask, a solder flux, a material for photofabrication, a material for a solar cell, an optical switch, a material for an optical circuit, a light sensor, a semiconductor-encapsulating agent, a coating for electronic parts, an encapsulating agent for electronic parts, a material for cultivating plants, a material for repairing trees, a material for road lines, a material for a greenhouse, a photodegradable plastic, an antibacterial plastic, a material for a sheet, a composite material (e.g., a composite material of glass fiber and other auxiliaries), an optical adhesive, an adhesive for optical discs (cD, DVD, blue laser discs, etc.), a material for powder coating, a material for aqueous coating, a refractive index adjuster, an encapsulating agent for LED, a potting agent, spectacle lenses, optical materials including lenses and prisms, a material for optical communication, a coating for optical fibers, a DNA chip, a material for preservation of plant seeds, a material for preservation of samples, a micro machine, sports goods, toys, automobile parts, putty for repairing automobiles, marine parts, aircraft parts, space materials, an encapsulating agent for liquid crystals, an adhesive for liquid crystal parts, an encapsulating agent and adhesive related to organic EL materials (pigments), an encapsulating agent and adhesive for pigment inductive solar cells, an adhesive for photosensitive optical discs, an adhesive for optical switches, a dental filling, a material for dental modeling, artificial teeth, an adhesive for teeth, an adhesive for biological materials, a plaster cast, a material for making an artificial arm and leg, an adhesive for bones, an adhesive for nails, an adhesive for hair, an adhesive and repairing agent for medical apparatus, bandage stoppers, a hemostatic agent, a material for fixation during an operation, a material for packaging drugs, a material for packing drugs, a material for printing on a package for tablets and a medicine box, name labels for drugs, a preservative for drugs, an adhesive for bandages, a material for a package of health foods and a material for printing on the package, a material for a package of beverages and a material for printing on the package, a material for immobilizing drugs, a material for packing agricultural chemicals, a preservative for agricultural chemicals, an agent for applying an insecticide, an agent for applying a repellent, an agent for preventing insects from scattering, an insect-catching agent, a material for packing a fertilizer, a material for preparing a DNA chip, an adhesive for a DNA chip, a preservative for DNA analysis, a material for a package of a diagnostic agent and a material for printing on the package, a preservative for biological drugs, a material for immobilizing fungi, a material for preserving fungi and viruses, a material for trapping fungi and viruses, a material for storing genes, a material for preserving seeds, a material for preserving biological samples, a material for preparing samples, a material for preserving electrophoresis results, a material for fixing a sample for microscopy, a repairing agent for trees, a fixing agent of plants and trees, a sustained-release agent for perfumes, a sustained-release agent for aromatics, a sustained-release agent for antiseptics, a sustained-release agent for microbicides, a sustained-release agent for insecticides, a sustained-release agent for odor-eliminating agents, a sustained-release agent for antirust agents, a sustained-release agent for fertilizers, a sustained-release agent for agricultural chemicals, a sustained-release agent for repellents, a material for DDS (drug delivery system), a material for (nano)imprinting technology, etc.

As cured products obtained by curing the resin composition or adhesive of the present invention by light irradiation are colorless or little colored, they are useful in fields where the color is important or for the purpose of looking into the inside. By use of this property, the resin composition or adhesive of the present invention can be used, for example, for preparation of dental fillings, color samples, and samples for observation of small animals, insects, fungi, etc.

With respect to the use as dental materials, visible light-curable photopolymerization initiators [e.g., camphorquinone and bis(2,4,6-trimethylbenzoyl)phenylphosphine oxide] are used to avoid the problems such as phototoxicy to human body and amblyopia. However, they have the problem of coloring (yellow), and there is no satisfactory material available for the esthetic treatment in which the beauty of appearance is a dominant factor. An irradiation equipment using blue LED (470±20 nm) has been put to practical use, but there are few photopolymerization initiators suitable for it and they are not satisfactory with respect to the curing ability. Further, when the inside of a cured product is not sufficiently cured, a patient may possibly swallow the resin or the filling may come out due to the insufficient adhesiveness. As the resin composition of the present invention is little colored, has high curability by visible light and can be sufficiently cured by using a dental irradiator, it is sufficiently useful for the esthetic treatment. Even when a dental filling (resin) comprising a filler is cured, the inside can be well cured.

Further, the resin composition or adhesive of the present invention having a high photosensitivity to visible light is excellent in ability of curing inside or curing a thick product and can be used as optical materials (e.g., potting materials and materials for lenses) and for the purposes of preserving samples, blocking the air, and the like. As to the use for constructive and architectural purposes, the resin composition or adhesive of the present invention is capable of curing the inside of a narrow pipe or can within the reach of light and therefore is useful for adhesion and repair of the inside of a earthenware pipe, a sewer pipe, a wall, a prop, etc. When the surface of a pipe is glossy, a part beyond a bend or the reach of hand in the pipe can be adhered and repaired by passing the adhesive of the present invention through a tube or the like and allowing visible light to reflect on the glossy surface of the pipe to reach the target part. It is difficult to transmit UV light for a long distance, but visible light can be transmitted using optical fibers and optical waveguides. Therefore, the resin composition or adhesive of the present invention can be used not only for adhesion of optical fibers themselves, but also for curing, adhesion and repairing of a part ahead thereof, and the use of optical fibers enables curing in the dark.

When the resin composition of the present invention is used for coating, it can be applied, for example, by dipping, brushing, spraying or roll-coating. The film thickness of and the kind of substrate material for coating with the resin composition of the present invention are selected according to the field of application. Examples of the appropriate substrate materials for recording photograph information are polyester, films of cellulose acetate, plastics, and resin coated papers. An example of the substrate material for offset printing plates is specifically treated aluminum, and an example of the substrate material for producing a printed circuit is copper-coated laminate.

By putting the resin composition or adhesive of the present invention between glass plates and curing it, a functional glass plate can be produced. For example, when the resin composition or adhesive of the present invention is put between glass plates and light cured, the cured product is formed into a film and the glass plates become a laminated glass suitable for crime prevention or glass scattering prevention. Similarly, a UV-cut glass plate is obtained by putting the resin composition or adhesive of the present invention comprising UVA between glass plates and light curing it; a colored glass plate is obtained by putting the resin composition or adhesive of the present invention comprising a pigment between glass plates and light curing it; a polarized glass plate is obtained by putting the resin composition or adhesive of the present invention comprising an asymmetric compound between glass plates and light curing it; a temperature-sensitive glass plate is obtained by putting the resin composition or adhesive of the present invention comprising a temperature-sensitive material between glass plates and light curing it; and a magnetic glass plate is obtained by putting the resin composition or adhesive of the present invention comprising magnetic powder between glass plates and light curing it.

Further, the resin composition of the present invention can be used as a coating material, for example, for coating of sheets and tubes, metal coating of a can or a cap of a bottle, coating of floors and walls, and coating of papers such as labels, cards, record jackets, cD and DVD jackets, milk packs, beverage packs, paper cups, cups for instant noodles, calendars, posters, business forms, record jackets and book covers.

The resin composition of the present invention can also be applied to preparation of an image or information carrier. For example, an image or information carrier can be prepared by coating the resin composition of the present invention, usually, on a substrate material to form a coating film, irradiating light through a photomask and treating the nonirradiated side with a solvent for removal.

The resin composition of the present invention can also form a coating film by electrodeposition onto metals. The light-irradiated part of the coating film, which becomes a cross-linked polymer, becomes insoluble in an alkaline solution and remains on the substrate material. When the remaining part is appropriately colored, a visible image is produced. When the substrate material is a vapor-deposited metal film, it is possible, after the light irradiation and development, to remove the metal from the nonirradiated side by etching or to increase the film thickness by zinc plating. In the above manner, a printed circuit plate can be produced.

The resin composition of the present invention, which is particularly suited for curing paints comprising a pigment and can cure a thick coating film, can also be used for producing a printing plate or a composite material.

The resin composition of the present invention can also be used as a resin for nanoimprint. The resin composition of the present invention can be employed mainly as a light-curable resin for optical nanoimprint technology or nanoimprint technology using both light and heat together.

The nanoimprint apparatus used in the present invention may be prepared by equipping an existing apparatus with, as a light source, a laser of the above near-ultraviolet light or visible light, or a blue light emitting diode (LED), or may be a newly designed apparatus utilizing visible light as a light source. It may also be an apparatus prepared by equipping an existing UV-curing apparatus with, at a light source, a UV absorbing plate or a UV-cut film, or an apparatus using, as a light source, sunlight and indoor illumination collected with a lens. By setting up a reflection plate on the upper surface and/or lower surface, pattern transfer over a wide area is possible by to-and-fro of the light.

It is desirable that the apparatus having a light source is equipped with a chamber which is vacuum or capable of gas substitution. However, as visible light irradiation, unlike UV irradiation, does not generate ozone, the chamber is not essential. Use of visible light irradiation is also advantageous for operators, because visible light irradiation, unlike UV irradiation, has no phototoxicity and no risk of amblyopia and ozone toxicity.

Of visible irradiation apparatus, a blue laser and an LED irradiation apparatus are capable of miniaturization and have such advantages as energy cut, long use of a light source and no phototoxicity to human body, and therefore are expected as an apparatus for general-purpose use.

As visible light penetrates a plastic material, when visible light is used as a light source, the resin composition of the present invention can be cured through a plastic substrate or film, and so, pattern transfer by nanoimprinting can be carried out. Even in the case of a high aspect ratio pattern or a resin comprising a UV absorber or a pigment, precise pattern transfer can be achieved. Further, release can be carried out without contaminating a formed pattern because the peripheral resin portion is cured by diffused light, and so a large pattern can be obtained and the production efficiency is enhanced.

A method of pattern transfer using the resin for nanoimprint of the present invention is illustrated below.

By using photolithography technique, the resin composition of the present invention is exposed on a quartz plate or a sapphire plate via a mask containing a desired pattern and etching is carried out to fabricate a mold having a rugged pattern. The mold materials are not limited to quarts or sapphire as long as they are materials which the wavelength for the light curing can permeate. As the resin composition of the present invention can be cured by visible light, more inexpensive plastics can be used as the mold materials. When a substrate is transparent, pattern transfer is possible by irradiating light from the side of the substrate even if an opaque mold is used.

The resin composition of the present invention is coated or spin-coated on a substrate of an inorganic or organic material such as silicon or glass. Generally, as the resin is solvent-free, the operation proceeds to the next step. However, when the resin composition contains a solvent or the like, drying by heating or under reduced pressure is carried out. Then, a mold is pressed on the resin coated on the substrate for press bonding, whereby the resin goes into the recesses and is formed into a shape corresponding to the rugged pattern of the mold pattern. A high pressure is not necessary for press bonding and the needed pressure is about 1 to 150 N. The light is irradiated usually from the side of the mold under this condition. When the substrate is transparent, irradiation from the side of the substrate is also possible.

Multi-layer transfer is a method in which a pattern is transferred on a pattern substrate obtained by thermal nanoimprint or photo nanoimprint. Use of the resin composition of the present invention widens the choice of substrate and mold materials and enables precise pattern transfer even when the resin layer becomes thick and a resin contains an additive. For example, use of resins different in color, refractive index, hydrophilicity, hydrophobicity, glass transition point, electric conductivity, light transmittance, or the like can impart a function according to the purpose. Specifically, use of resins different in refractive index can produce an optical waveguide and an optical coupler, and use of resins different in hydrophilicity can produce a micro passage and an electrophoresis passage.

When pattern transfer is performed, a film or a foil may be put between a mold and a resin according to the purpose. Examples of materials to be put between the mold and the resin are a polyimide film, an electrically conductive film, a gold foil, a copper foil and an aluminum foil. When an electrically conductive foil is used, the obtained product can be used as an electric circuit and a distribution board after removing the conductive foil from the surface of protruding portions.

A film or a microstructure body having a rugged surface can be produced using a mold on both or one of the upper and lower surfaces. By using a visible light curable resin, precise pattern transfer can be performed even when a resin layer or a film layer becomes thick.

The resin composition of the present invention is capable of producing various structures by combining molds and the above techniques and can be cured by visible light, and therefore can accurately produce or duplicate replicas of microstructures or micro parts.

A functional microstructure can be produced by processing the transfer pattern (the substrate may be included) or the cured product obtained by the above techniques by such methods as etching. The methods for processing include, in addition to chemical etching, electron beam exposure, laser cutting, heat treatment and electric treatment. A processed microstructure can be used, for example, as a semi conductor chip or an LSI chip after undergoing the etching process, or can be used as optical elements or a functional film by cutting out.

The materials of substrates which can be coated with the resin composition of the present invention are not specifically limited, and include, for example, glass, oxide film-coated glass (ITO: indium titanium oxide-coated glass, etc.), metals (aluminum, gold, silver, copper, iron, brass plate, tin plate, etc.), plastics (polycarbonate, acryl, PET (polyethylene terephthalate) and ABS (acrylonitrile-butylene-styrene copolymer) resin plates, etc.), films (polyimide, vinyl chloride, polystyrene and saran resin films, etc.), papers (office papers, posters, drawing papers, etc.), building materials (slates, blocks, bricks, gypsum boards, etc.), porcelain (pottery, ceramics, tiles, etc.), woods and parts of human body (skin, bones, nails, hair, body hair, cells, blood vessels, etc.).

When a substrate is a material which reflects light, the visible light reaches the inside, reflects on the substrate and reaches every part of the pattern, which enhances the curing efficiency and enables curing of complicated places. When a substrate is transparent or half-transparent, light permeates the substrate and so the light can be irradiated from the side of the substrate, which can enhance the curing efficiency.

The mold used in the present invention which is cured by light is preferably a material which light permeates, and it is more preferred that both or one of the substrates to be adhered are transparent or half-transparent. When the adhered layer is thick, curing can be carried out by irradiating the light from between the adherends. Examples of the materials for transparent molds are quartz, sapphire, glass, diamond, plastic materials [polyethylene, polypropylene, polyoxymethylene, polyvinyl chloride, methyl polymethacrylate, polyamide, polystyrene, polyethylene fluoride, polycarbonate, polyimide, polyphenylene oxide, polyurethane, polyethylene terephthalate, polyphenylene isophthalamide, polyacrylonitrile (acryl resin), an epoxy resin, a silicon resin, a melamine resin, a polyester resin, a saran resin, etc.] and other plastic resins. When an opaque mold is used, it is preferable to use a transparent material for a substrate. Examples of the materials for opaque molds are silicon, metals (aluminum, iron, titanium, silver, gold, copper, platinum, niobium, lead, tin, zinc, cobalt, nickel, chromium, indium, tantalum, zirconium, molybdenum, tungsten, bismuth, cadmium, magnesium, etc.), metal oxides (alumina, iron oxide, copper oxide, titanium oxide, zirconium oxide, etc.), steels (carbon steel, stainless steel, nickel steel, molybdenum steel, etc.), alloys (bronze, brass, brass, cupronickel, duralumin, monel, cupronickel, etc.), plastic materials (same as the above transparent mold materials), papers, building materials (wall materials, bricks, blocks, tiles, etc.) and stones (granite, mica, marble, basalt, etc.). Mold materials include not only artificial products, but also natural products and biological materials, such as fingerprints, nails, skin, blood vessels, hair, body hair, bones, cartilages, insects, crustaceans, teeth (human teeth, cow teeth, pig teeth, shark teeth, etc.), feather, beaks, tusks (elephant tusks, tusks of marine animals, etc.), turtle shells, minerals (sand, chesil, crystallized stones, opal, turquoise, etc.), amber, plants (teeth, stems, roots, fruits, flowers, seeds, fruits, bulbs, barks, etc.), microorganisms (cladoceran, paramecium, etc.), algae, corals, fins, scales and gills.

The transferred pattern or cured product obtained from the resin composition of the present invention can be used as a semiconductor chip, LSI, a printed electronic circuit, a material for micro parts, a material for molecular devices, a micro machine, a printing plate, a material for production of a printing mask, a material for production of a mold, a material for a photo-reproduction process, a material for a picture-recording process, a material for optical recording, a material for reproduction of organs, a material for plaster casts, a material for designing, a material for designing, a material for making a model of a small apparatus, a material for making a simulation model, a material for FPD (flat panel display), LcD (liquid crystal display), a material for a color filter, a material for organic TFT, a material for a functional film, an electrically conductive film, a reflection-preventing film, a surface-coating material, a prism sheet, an optical element, a photonic element, photonic crystals, a honeycomb structure, an optical circuit, a lens, a prism, a fresnel lens, a hologram, a microlens array, a material for optical communication, an optical waveguide, optical fibers, an optical coupler module, a material for a solar cell, an optical switch, a material for an optical circuit, a material for hologram, a material for photofabrication, a light sensor, an optical morphology, an electronic paper, LED (light emitting diode), a material for organic EL, a material for a display, an ink, a material for an ink jet printer, a printing plate, a stamp pad, a typing keyboard, a material for a stamp, dental materials, a medium for cells, a medium for bacteria, a medium for nerves, a container for the preservation of organs, an electrophoresis apparatus, a micro passage, a biochip, a DNA chip, DDS (drug delivery system), a diagnostic kit, a plant cultivation kit, a fingerprint verification system, a fingerprint-duplicating system, a vein verification system, an iris identification system, a material for duplicating teeth and fangs, a material for duplicating nails, a material for duplicating hair, a material for preparing a sample, automobile parts, marine parts, aircraft parts, space materials, etc.

The present invention is described in more detail by referring to the following synthesis examples, examples and comparative examples.

### (Synthesis Example 1)

### Synthesis of 9-Phenyl-9-Phosphafluorene (compound 2)

Tetrahydrofuran (200 mL) and diethylamine (90 mL) were put into a flask equipped with a reflux condenser, and the mixture was cooled to 0ºc in an atmosphere of nitrogen, followed by dropwise addition of an n-butyl lithium/n-hexane solution (1.6 mol/L, 500 mL; Kanto chemical co., Inc.) over 4 hours. Then, tetraphenylphosphonium bromide (163 g; Hokko chemical Industry co., Ltd.) was gradually added and the resulting mixture was stirred at 0ºc for one hour and at room temperature for 3 hours. After the reaction mixture was cooled to 0ºc, water (100 mL) and then hydrochloric acid (2M, 500 mL) were gradually added. The reaction product was extracted with ether (500 mL), and the organic layer was washed with a saturated aqueous solution of sodium chloride (500 mL) and dried over anhydrous magnesium sulfate. After filtration, the product was concentrated using a rotary evaporator and recrystallized from toluene/methanol. The crystals were collected and dried under reduced pressure to obtain compound 2 as 92.5 g of white crystals (yield: 91%).

### (Synthesis Example 2)

### Synthesis of 9-(2,4,6-Trimethylbenzoyl)-9-Oxo-9-Phosphafluorene (compound 1-1)

9-Phenyl-9-phosphafluorene (650 mg, 2.5 mmol), tetrahydrofuran (7.5 mL) and lithium metal (45 mg, 15 mmol) were put into a flask equipped with a reflux condenser, and the mixture was stirred at room temperature for 3 hours in an atmosphere of argon. After the reaction solution was transferred into a flask equipped with a reflux condenser (atmosphere of argon, room temperature), tert-butyl chloride (0.27 mL, 2.5 mmol) was added thereto, and the resulting mixture was stirred for 10 minutes with heating at 60ºc, followed by cooling to room temperature. The resulting mixture was added dropwise to a flask (atomosphere of argon) containing a tetrahydrofuran solution (7.5 mL) of 2,4,6-trimethylbenzoyl chloride (460 mg, 2.5 mmol) cooled to -78ºc. The reaction mixture was stirred at -78ºc for 15 minutes, and oxygen gas (1 atm) was blown into the flask. Then the mixture was stirred for one hour and the temperature was raised to room temperature. After the reaction mixture was concentrated, the residue was diluted with dichloromethane (5 mL) and purified by separation with silica gel column chromatography (n-hexane and ether, successively). The fraction eluted with ether was concentrated and the residue was recrystallized from dichloromethane/n-hexane solvent mixture. The crystals obtained by filtration were dried under reduced pressure to obtain compound 1-1 (yield: 54%, 430 mg). 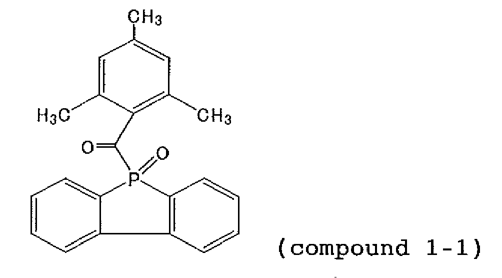 (compound 1-1)
¹H NMR(CDCL₃) :
d2.10(s,6H),2.24(s,3H),6.85(s,2H),7.23(Dt,J=3.69,7.57Hz,2H),7.64(t, J=7.57Hz,2H),7.75(DD,J=7.57,8.01Hz,2H),7.88(DD,J=2.93,7.57Hz,2H)
¹³C NMR(CDCL₃) :
d21.65,25.56,121.19,130.02(D,J=10.94Hz),129.10,130.03(D,J=97.44Hz),13 1.09(D,J=9.32Hz),134.59,134.64(D,
J=11.77Hz),137.15(D,J=43.00Hz),140.75,143.04(D,J=21.13Hz),216.22(D,J= 73.06Hz)
³¹P NMR(CDCL₃) : d+29.12
IR (KBR,cm⁻¹) : ν2973.7,1608.3,1438.6,1176.4,717.4,570.8
Elemental analysis:
calcd. for c₂₂H₁₉O₂P: c, 76.29 ; H,5.33 ; O,9.24
Found : c,76.12; H,5.64; O,9.09

### (Synthesis Example 3)

### Synthesis of 9-Oxo-9-(4-Toluoyl)-9-Phosphafluorene (compound 1-2)

Reaction was carried out in the same manner as in Synthesis Example 2 except that p-toluoyl chloride was used in place of 2,4,6-trimathylbanzoyl chloride, and the reaction solution was concentrated. The residue was diluted with dichloromethane (5 mL) and purified by separation with silica gel column chromatography (diethyl ether:n-hexane=5:1). The eluate was concentrated and the residue was recrystallized from dichloromethane/n-hexane solvent mixture. The crystals obtained by filtration were dried under reduced pressure to obtain compound 1-2 (yield: 52%, 413 mg). 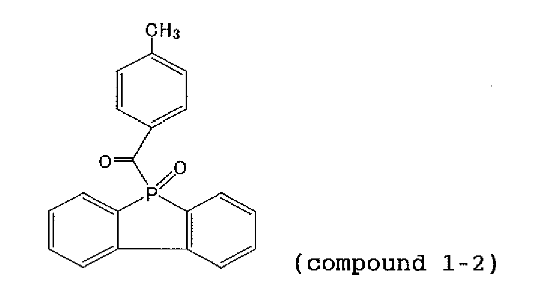 (compound 1-2)
¹H NMR(CDCL₃,PPm) : d2.49(s,3H),7.34.-7.37(m,2H),7.36(D,J=7.74Hz,2H),7.48(Dt,J=0.98,7.47Hz,2H),7.74(DD,J=4. 85,7.47Hz,2H), 7.92(D,J=7.74Hz,2H),8.01(DD,J=2.11,8.18Hz,2H)
¹³c NMR (CDCL₃,PPm) :
d23.82,121.78,128.07(D,J=7.77Hz),128.96(D,J=.8.98Hz),129.31, 129.82,130.46(D,J=28.56Hz),131.58(D,J=19.62 Hz),135.99,137.51(D,J=36.3Hz),144.48(D,J=79.78Hz),210.35(D.J=71.53Hz)
³¹P NMR(CDCL₃,PPm):d+17.11
IR (KBR,cm^{.-1}) : ν2929.3,1641.3.1598.7,1174.4,746.3
F A B .-MS m/z (relative intensity) : 519 [8%, (M+C₁₂H₉PO)⁺] ,319 [43%,(M+H)⁺],119(100%,c₁₂H₉PO⁺)

### (Synthesis Example 4)

### Synthesis of 9-(p-Anisoyl)-9-Oxo-9-Phosphafluorene (compound 1-3)

The same procedure as in Synthesis Example 2 was repeated except that p-anisoyl chloride was used in place of 2,4,6-trimethylbenzoyl chloride to obtain compound 1-3 (7%, 0.60 g). 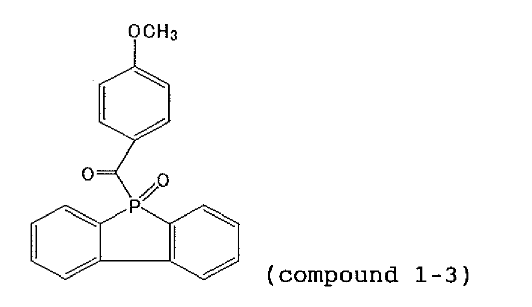 (compound 1-3)
¹H NMR(CDCL₃,PPm):
d3.82(s,3H),7.01(D,J=8.67Hz,2H),7.33(DDt,J=1.07,3.17,7.43Hz,2H),7.44( Dt,J=1.19,7.43Hz,2H),7.72(DD,J=4.71,7.43Hz,2H),7.89(D,J=7.43Hz,2H),8. 07.-8.17(m,2H).
¹³C NMR(CDCL₃,PPm):
d56.12,114.35,121.71,128.31(D,J=7.77Hz),129.55,131.51(D,J=9.74Hz),131 .78(D,J=19.70Hz),133.32(D,J=47.93Hz),136.54(D,J=1.43Hz),144.26(D,J=2. 34Hz),164.39,208.73(D,J=37.59Hz)
³¹P NMR(CDCL₃,PPm) :d+15.86
IR (KBR,cm^{.-1}) : ν3073.9,2838.7,1596.8,1504.2,1186.0

### (Synthesis Example 5)

### Synthesis of 9-(2,6-Dimethoxybenzoyl)-9-Oxo-9-Phosphafluorene (compound 1-4)

The same procedure as in Synthesis Example 2 was repeated except that 2,6-dimethoxybenzoyl chloride was used in place of 2,4,6-trimethylbenzoyl chloride to obtain compound 1-4 (46%, 4.1 g). 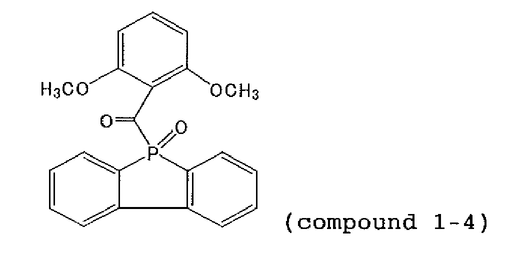 (compound 1-4)
¹H NMR(CDCL₃,PPm) : d3.83(s,6H),6.53(D,J=8.40Hz,2H),7.31(t,J=8.40Hz,1H),7.28.-7.44(m,2H),7.59(Dt,J=1.23,8.22Hz,2H),7.76.-7.80(m,2H),7.85(D,J=8.22Hz,2H)
¹³C NMR (CDCL₃, PPm) : d56.71,104.72,117.26(D,J=9.89Hz),129.67(D,J=11.25Hz),131.15(D,J=10.03 Hz),131.20(D,J=98.80Hz),133.79,134.30(D,J=2.05Hz),143.36(D,J=20.30Hz) ,158.48,209.42(D,J=84.53Hz)
³¹P NMR(CDCL₃,PPm):d+25.92
IR (KBR,cm⁻¹): ν3066.2,2834.8,1687.9,1592.9,1207.2

### (Synthesis Example 6)

### Synthesis of 9-(1-Naphthoyl)-9-Oxo-9-Phosphafluorene (compound 1-5)

The same procedure as in Synthesis Example 2 was repeated except that 1-naphthoyl chloride was used in place of 2,4,6-trimethylbenzoyl chloride to obtain compound 1-5 (51%, 450 mg). 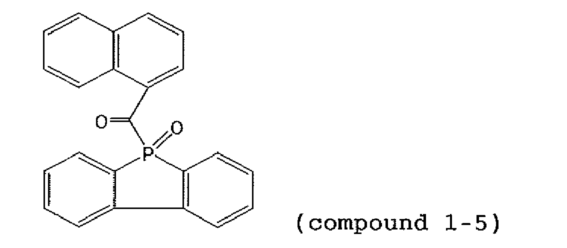 (compound 1-5)
¹H NMR(CDCL₃,PPm):
d7.23(D,J=3,12Hz,1H),7.27(D,J=1.25Hz,1H),7.31(D,J=1.08Hz,1H),7.34(Dt, J=3.03,11.95Hz,2H),7.40.-7.48(m,2H),7.56(DD,J=4.57,7.38Hz,2H),7.68(t,J=7.45Hz,1H),7.85(D,J=8.2 2Hz,2H),7.89(D,J=7.82Hz,1H),8.02(DD,J=5.96,8.28Hz,1H),8.45.-8.49(m,2H)
IR(KBR,cm^{.-1}) : ν1641.1,1216.0

### (Synthesis Example 7)

### Synthesis of 9-Oxo-9-(2-Toluoyl)-9-Phosphaphosphafluorene (compound 1-6)

The same procedure as in Synthesis Example 2 was repeated except that o-toluoyl chloride was used in place of 2,4,6-trimethylbenzoyl chloride to obtain compound 1-6 (27%, 212 mg). 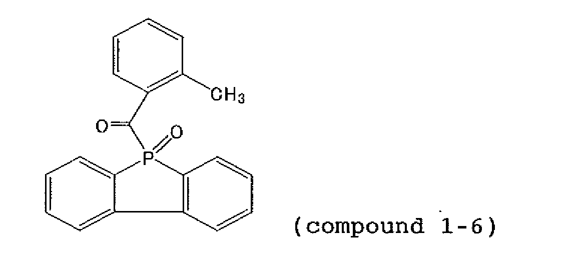 (compound 1-6)
¹H NMR(CDCL₃,PPm) : d1.9(s,3H),7.14.-7.15(m,1H),7.29(DDt,J=0.85,3.03,7.58Hz,2H),7.36.-7.42(m,2H),7.42(Dt,J=0.85,7.58Hz,2H),7.50(DD,J=4.64,7.58Hz,2H),7.88(D ,J=7.58Hz,2H),8.06.-8.10(m,1H)
¹³C NMR (CDCL₃,PPm) : d21.59,121.62,125.83,128.15(D,J=7.67Hz),129.13,129.31,130.51(D,J=93.2 9Hz),131.40(D,J=19.50Hz),131.78,131.80,134.43,140.25(D,J=32.57Hz),143 .62(D,J=2.43Hz),215.08(D,J=48.91Hz)
³¹P NMR(CDCL₃,PPm ):d+21.87
IR (KBR,cm^{.-1}):ν3058.6,1646.9,1436.7,1189.9,894.9,748.2,644.1

### (Synthesis Example 8)

### Synthesis of 9-Oxo-9-(4-Pentyloxybenzoyl)-9-Phosphafluorene (compound 1-7)

9-Phenyl-9-phosphafluorene (6.50 g, 25 mmol), tetrahydrofuran (75 mL) and lithium metal (450 mg, 150 mmol) were put into a flask, and the mixture was stirred at room temperature for 3 hours in an atmosphere of argon. After the reaction solution was transferred into a flask equipped with a reflux condenser (atmosphere of argon, room temperature), tert-butyl chloride (2.7 mL, 25 mmol) was added thereto, and the resulting mixture was stirred at 60ºc for 15 minutes, followed by cooling to room temperature. The resulting mixture was added dropwise to a flask (atmosphere of argon) containing a tetrahydrofuran solution (75 mL) of p-pentyloxybenzoyl chloride (5.67 g, 25 mmol) cooled to -78ºc. Then the reaction mixture was stirred at -78Qc for 60 minutes and oxygen was introduced into the flask containing the obtained reaction solution (-78ºc), followed by stirring at this temperature for one hour. The reaction mixture was concentrated, while raising the temperature to room temperature, and the obtained residue was diluted with 7 mL of dichloromethane and filtered. After the filtrate was concentrated, the residue was recrystallized from dichloromethane/n-hexane. The crystals obtained by filtration were dried under reduced pressure to obtain compound 1-7 (35%, 3.43 g). 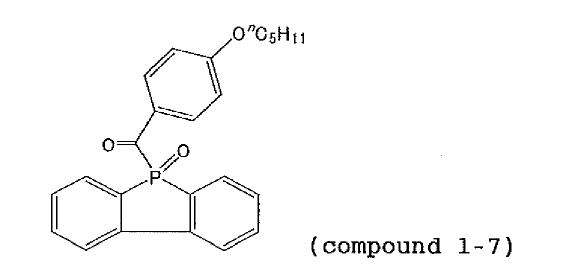 (compound 1-7)
¹H NMR(CDCL₃,PPm) : d0.94(t,J=7.01Hz,3H),1.34.-1.51(m,4H),1.78,-1.87(m,2H),4.04(t,J=6.54Hz,2H),6.99(D,J=8.80Hz,2H),7.33(DDt,J=0.97,3. 02,7.44Hz,2H),7.44(Dt,J=0.97,7.44Hz,2H),7.72(DD,J=4.71,7.44Hz,2H),7.8 9(D,J=7.44Hz,2H),8.09(DD,J=6.43,8.80Hz,2H)
¹³C NMR(CDCL₃,PPm) : d13.99,22.42,28.12,28.78,68.38,114.32,121.36,127.60(D,J=7.65Hz),128.8 2,130.84(D,J=9.66Hz),131.09(D,J=19.78Hz),132.43(D,J=37.77Hz),135.97(D ,J=1.76Hz),143.58(D,J=2.41Hz),163.51(D,J=1.64Hz),207.44(D,J=47.87Hz)
³¹P NMR(CDCL₃,PPm): d+12.58
IR (KBR,cm^{.-1}) : ν3056.6,2954.4,2869.6,1641.1,1160.9
Elemental analysis:
calcd. for c₂₃H₁₇O₂P : c,73.87; H,5.94
found : c,73.61 ; H,6.05

### (Synthesis Example 9)

### Synthesis of 9-(4-Hexylbenzoyl)-9-Oxo-9-Phosphafluorene (compound 1-8)

9-Phenyl-9-phosphafluorene (2.60 g, 10 mmol), tetrahydrofuran (30 mL) and lithium metal (180 mg, 60 mmol) were put into a flask, and the mixture was stirred at room temperature for 3 hours in an atmosphere of argon. After the reaction solution was transferred into a flask equipped with a reflux condenser (atmosphere of argon, room temperature), tert-butyl chloride (1.08 mL, 10 mmol) was added thereto, and the resulting mixture was stirred at 60ºc for 15 minutes, followed by cooling to room temperature. The resulting mixture was added dropwise to a flask (atmosphere of argon) containing a tetrahydrofuran solution (30 mL) of p-hexylbenzoyl chloride (5.62 g, 10 mmol) cooled to -78ºc. Then the reaction mixture was stirred at -78ºc for 60 minutes and air was blown into the flask containing the obtained reaction solution (-78ºc), followed by stirring at this temperature for one hour. The reaction mixture was concentrated, while raising the temperature to room temperature, and the obtained residue was diluted with 7 mL of dichloromethane and filtered. After the filtrate was concentrated, the residue was recrystallized from dichloromethane/n-hexane. The crystals obtained by filtration were dried under reduced pressure to obtain compound 1-8 (42%, 1.63 g). 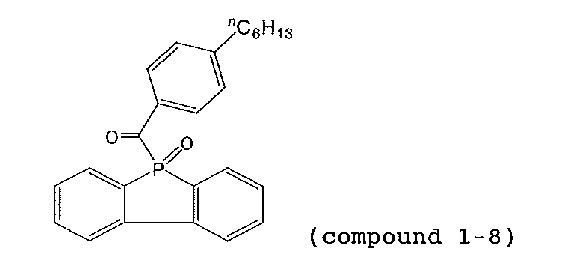 (compound 1-8)
¹H NMR(CDCL₃,PPm) : d0.89(t,J=6.83Hz,3H),1.32.-1.37(m,6H),1.61.-1.67(m,2H),2.69(t,J=7.68Hz,2H),7.30.-7.34(m,4H),7.44(Dt,J=0.92,7.63Hz,2H),7.71(DD,J=4.76,7.32Hz,2H),7.88(D ,J=7.63Hz,2H),7.99(DD,J=2.13,8.20Hz,2H)
¹³C NMR(CDCL₃,PPm) : d14.06,22.56,28.91,30.97,31.63,36.11,121.36(D,J=0.72Hz),127.63(D,J=7. 70Hz),128.54(D,J=8.98Hz),128.73,128.88,131.16(D,J=19.64Hz),135.59(D,J =1.62Hz),137.25(D,J=36.29Hz),143.50(D,J=2.53Hz),149.51(D,J=1.09Hz),20 9.43(D,J=48.81Hz)
³¹P NMR(CDCL₃,PPm) : d+13.97
IR (KBR,cm^{.-1}) : ν3054.7,2956.3,2917.8,2854.1,1645.0,1172.5

### (Synthesis Example 10)

### Synthesis of 9-Oxo-9-(2-Tenoyl)-9-Phosphafluorene (compound 1-9)

The same procedure as in Synthesis Example 8 (synthesis of compound 1-7) was repeated except that 2-tenoyl chloride was used in place of p-pentyloxybenzoyl chloride to obtain compound 1-9 (45%, 3.33 g). 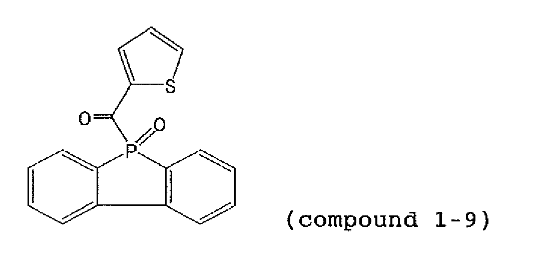 (compound 1-9)
¹H NMR(CDCL₃,PPm) : d7.25(DD,J=3.79,4.86Hz,1H),7.37(DDt,J=0.98,2.99.7.46Hz.2H),7.48(Dt,J= 0.98,7.46Hz,2H),7.71(DDD,J=1.09,2.10,4.86Hz,1H),7.78(DD,J=5.07,7.46Hz ,2H),7.91(D,J=7.46Hz,2H),8.23(DDD,J=0.94,1.09,3.79Hz,1H)
¹³C NMR(CDCL₃,PPm) : d121.46,127.78(D,J=7.86Hz),128.02,129.22,131.16(D,J=19.64Hz),133.87(D ,J=9.53Hz,1c),134.51(D,J=1.92Hz),135.47(D,J=2.89Hz),144.06(D,J=2.53Hz ),145.94(D,J-45.37Hz),200.07(D,J=45.85Hz)
³¹P NMR(CDCL₃,PPm) : d+11.92
IR (KBR,cm⁻¹) : ν3081.7,1619.9,1199.5,939.2,858.2,798.4

### (Synthesis Example 11)

### Synthesis of 9-Oxo-9-[5-(2'-Thienyl)-2-Tenoyl]-9-Phosphafluorene (compound 1-10)

9-fhenyl-9-phosphafluorene (650 mg, 2.5 mmol), tetrahydrofuran (7.5 mL) and lithium metal (45 mg, 15 mmol) were put into a flask, and the mixture was stirred at room temperature for 3 hours in an atmosphere of argon. After the reaction solution was transferred into a flask equipped with a reflux condenser (atmosphere of argon, room temperature), tert-butyl chloride (0.27 mL, 2.5 mmol) was added thereto, and the resulting mixture was stirred at 60ºc for 15 minutes, followed by cooling to room temperature. The resulting mixture was added dropwise to a flask (atmosphere of argon) containing a tetrahydrofuran solution (7.5 mL) of 5-(2'-thienyl)-2-tenoyl chloride (572 mg, 10 mmol) cooled to -78ºc. Then the reaction mixture was stirred at -78ºc for 60 minutes and air was blown into the flask containing the obtained reaction solution (-78ºc), followed by stirring at this temperature for one hour. The reaction mixture was concentrated, while raising the temperature to room temperature, and the obtained residue was diluted with 7 mL of dichloromethane and filtered. After the filtrate was concentrated, the residue was recrystallized from dichloromethane/n-hexane. The crystals obtained by filtration were dried under reduced pressure to obtain compound 1-10 (44%, 428 mg). 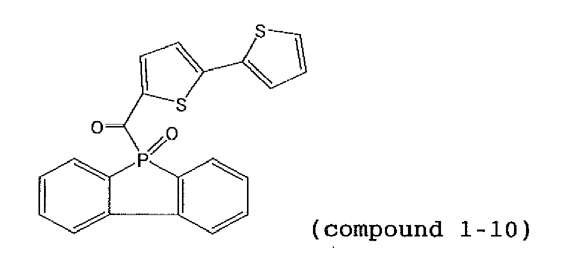 (compound 1-10)
¹H NMR(CDCL₃,PPm) : d7.03(DD,J=3.59,4.40Hz,1H).7.28.-7.33(m,3H),7.37(Dt,J=2.40,7.39Hz,2H),7.48(t,J=7.39Hz,2H),7.78(DD,J=6. 19,7.39Hz,2H),7.91(D,J=7.39Hz,2H),8.13(D,J=3.59Hz,1H)
¹³C NMR (CDCL₃,PPm) : d121.49,124.06,126.04,126.95,127.82(D,J=7.85Hz),128.30,129.25,131.06( D,J=20.76Hz),135.00(D,J=10.34Hz),135.65(D,J=2.57Hz),136.02,143.71(D,J =46.34Hz),144.14(D,J=1.89Hz).144.63,199.32(D,J=48.23Hz)
³¹P NMR(CDCL₃,PPm) : d+10.88
IR (KBR,cm^{.-1}) :ν3095.2,3062.4,1639.2,1442.5,1166.7,1079.9,756.0 Elemental analysis:
calcd. for c₂₁H₁₃O₂PS₂:c,64.27;H,3.34;S,16.34
Found : c,64.52 ; H, 3. 50 ; S,16.32

### (Test on Adhesion by Light curing)

The present invention is described in detail by referring to the following examples and comparative examples. These examples are not to be construed as limiting the scope of the present invention.

The abbreviations used in the following Tables 1 to 7 are explained below.
UA (urethane acrylate): polycarbonate-denatured urethane acrylate UN-9200A (Negami chemical Industrial co., Ltd.)
EA (epoxy acrylate): bisphenol A type-denatured epoxy diacrylate Ebecryl 3700 (Daicel-UcB co., Ltd.)
A-HD (1,6-hexanediol diacrylate): A-HD (Shin-Nakamura chemical co., Ltd.)
AcMO (acryloylmorpholine): AcMO (Kohjin co., Ltd.)
HEMA (2-hydroxyethyl methacrylate): (Tokyo chemical Industry co., Ltd.)
Et-cHDA (ethoxylated cyclohexanedimethanol diacrylate): NK Ester A-cHD-4E (Shin-Nakamura chemical co., Ltd.)
APG700 (polypropylene glycol diacrylate #700: NK Ester APG-700 (Shin-Nakamura chemical co., Ltd.)
AGP200 (tripropylene glycol diacrylate): NK Ester APG-200 (Shin-Nakamura chemical co., Ltd.)
Silane-E (γ-glycidoxypropyltrimethoxysilane): KBM-403 (Shin-Etsu chemical co., Ltd.)
Silane-A (γ-methacryloxypropyltrimethoxysilane): KBM-503 (Shin-Etsu chemical co., Ltd.)
MAPO (2,4,6-trimethylbenzoyl diphenylphosphine oxide): Lucirin TPO (BASF)
BAPO: bis(2,4,6-trimethylbenzoyl)phenylphosphine oxide: IRGAcURE 819 (BASF)
cQ (camphorquinone): (Wako Pure chemical Industries, Ltd.) EDMAB (ethyl p-dimethylaminobenzoate): KAYAcURE EPA (Nippon Kayaku co., Ltd.)
DETX-S (2,4-diethylthioxanthone): KAYAcURE DETX-S (Nippon Kayaku co., Ltd.)
IR651 (2,2-dimethoxy-1,2-diphenylethan-1-one): IRGAcURE 651 (ciba Specialty chemical corp.)
IR184 (1-hydroxycyclohexyl phenyl ketone): IRGAcURE 184 (ciba Specialty chemical corp.)
Titanium oxide (rutile type): (Wako Pure chemical Industries, Ltd.)
UVA (UV absorber): 2-(5-methyl-2-hydroxyphenyl)benzotriazole: TINUVIN P (ciba Specialty chemical corp.)

### Example 1

The components shown in Table 1 were mixed to obtain a resin composition. Mixing was carried out at 50ºc (the same shall apply to the following examples and comparative examples).

### Example 2

The components shown in Table 1 were mixed to obtain a resin composition.

### Example 3

The components shown in Table 1 were mixed to obtain a resin composition.

### (comparative Example 1)

The components shown in Table 1 were mixed to obtain a resin composition.

### (comparative Example 2)

The components shown in Table 1 were mixed to obtain a resin composition.

### Example 4

The components shown in Table 2 were mixed to obtain a resin composition.

### Example 5

The components shown in Table 2 were mixed to obtain a resin composition.

### (comparative Example 3)

The components shown in Table 2 were mixed to obtain a resin composition.

### (comparative Example 4)

The components shown in Table 2 were mixed to obtain a resin composition.

### (comparative Example 5)

The components shown in Table 2 were mixed to obtain a resin composition.

### Example 6

The components shown in Table 3 were mixed to obtain a resin composition.

### Example 7

The components shown in Table 3 were mixed to obtain a resin composition.

### Example 8

The components shown in Table 3 were mixed to obtain a resin composition.

### Example 9

The components shown in Table 3 were mixed to obtain a resin composition.

### (comparative Example 6)

The components shown in Table 3 were mixed to obtain a resin composition.

### (comparative Example 7)

The components shown in Table 3 were mixed to obtain a resin composition.

### Example 10

The components shown in Table 4 were mixed to obtain a resin composition.

### Example 11

The components shown in Table 4 were mixed to obtain a resin composition.

### Example 12

The components shown in Table 4 were mixed to obtain a resin composition.

### Example 13

The components shown in Table 4 were mixed to obtain a resin composition.

### (comparative Example 8)

The components shown in Table 4 were mixed to obtain a resin composition.

### (curing Ability Test)

The resin compositions obtained in Examples 1 to 13 and comparative Examples 1 to 8 were used in the following curing ability test.

### Test using polycarbonate plates:

Each of the resin compositions (0.15 g) was dropped on the center of a transparent polycarbonate plate [40 mm x 40 mm x 2 mm (thickness): hereinafter sometimes referred to as a polyca plate], and another polyca plate was put on the above plate to evenly spread the resin composition between them. The composition was cured by various kinds of lights, and the number of times of curing, the curing period and the yellowness index at the time when the two polyca plates were fixed to each other were recorded.

### Test using glass plates:

Each of the resin compositions (0.2 g) was dropped on the center of a transparent glass plate [50 mm x 50 mm x 2 mm (thickness)], and another glass plate was put on the above plate to evenly spread the resin composition between them. The composition was cured by various kinds of lights, and the number of times of curing, the curing period and the yellowness index at the time when the two glass plates were fixed to each other were recorded.

### [Lights]

SL: sunlight (fine weather: from 10:00 to 15:00)
UV: ultraviolet light
curing was carried out using a light source device for UV curing [metal halide lamp and high-pressure mercury lamp (Eyegraphics co., Ltd.), 400 mJ, 80 W/cm², belt speed: 5 m/min.]
VL-S: visible light (strong)
Each of the resin compositions was cured by using the above light source device for UV curing with a glass plate coated with a UV-cut film (transparent) being put on the resin composition to shield UV light. UV-cut film (transparent): Achilles Vinylus (transparent), thickness: 0.2 mm, wavelength: less than 370 nm, 100% cut; 400 nm, 60% cut; more than 530 nm, 10% cut (Achilles corp.)
VL-W: visible light (weak)
Each of the resin compositions was cured by using the above light source device for UV curing with a glass plate coated with a UV-cut film (smoke) being put on the resin composition to shield UV light.
UV-cut film (smoke): Achilles Vinylus (smoke), thickness: 0.2 mm, wavelength: less than 370 nm, 100% cut; 400 nm, 85% cut; 500 nm, 60% cut; 600 nm, 53% cut (Achilles corp.)

For the curing test, cured products having the film thickness of 80±10 µm (average thickness of 5 arbitrary points) were used. Three samples were prepared and the average data on the number of times, curing period and yellowness index are shown.

In the table, the curing period (minute) indicates the time required for the two plates to be fixed to each other by curing.

In the table, the number of times indicates the number of times the plates were passed through the light source device, and X indicates that curing was insufficient even after the plates were passed through the device 10 times.

The yellowness index was measured using a colorimeter (SE 2000, Nippon Denshoku Industries co., Ltd.). The measurement of the yellowness index (YI) of test subjects including the two test plates was carried out 3 times and the average values were recorded.

The results of the above evaluation are shown in Tables 1 to 4.

**Table 1**

| | | Ex. 1 | Ex. 2 | Ex. 3 | comp. Ex. 1 | comp. Ex. 2 |
|---|---|---|---|---|---|---|
| Resin comp. | UA | 50 | 50 | 50 | 50 | 50 |
| | A-HD | 40 | 40 | 40 | 40 | 40 |
| | AcMO | 10 | 10 | 10 | 10 | 10 |
| Initiator | compound 1-2 | 1 | | | | |
| | compound 1-7 | | 1 | 2 | | |
| | MAPO | | | | 2 | |
| | cQ | | | | | 2 |
| Polyca plate | SL, (minute) | 1 | 1 | 0.5 | 2 | 2 |
| | UV (no. of times) | 1 | 1 | 1 | 1 | 1 |
| | VL-S (no. of times) | 1 | 1 | 1 | 1 | 1 |
| | VL-W (no. of times) | 1 | 1 | 1 | 2 | 3 |
| Glass plate | SL (minute) YI | 0.5 0.3 | 0.5 0.2 | 0.5 0.2 | 1 1.1 | 1 8.5 |
| | UV (no. of times) YI | 1 0.2 | 1 0.2 | 1 0.2 | 1 0.2 | 1 8.6 |
| | VL-S (no. of times) YI | 1 0.2 | 1 0.2 | 1 0.2 | 1 0.2 | 1 7.1 |
| | VL-W (no. of times) | 1 | 1 | 1 | 2 | 2 |

| | | | | | |
|---|---|---|---|---|---|
| YI | 0.3 | 0.3 | 0.5 | 0.5 | 8.3 |

**Table 2**

| | | Ex. 4 | Ex. 5 | comp. Ex. 3 | comp. Ex. 4 | comp. Ex. 5 |
|---|---|---|---|---|---|---|
| Resin comp. | UA | 30 | 30 | 30 | 30 | 30 |
| | Et-cHDA | 40 | 40 | 40 | 40 | 40 |
| | HEMA | 30 | 30 | 30 | 30 | 30 |
| Initiator | compound 1-2 | 1 | | | | |
| | compound 1-7 | | 1 | | | |
| | MAPO | | | 1 | | |
| | BAPO | | | | 1 | |
| | IR651 | | | | | 1 |
| Polyca plate | SL (minute) | 1 | 1 | 5 | 2 | 20 |
| | UV (no. of times) | 1 | 1 | 1 | 1 | 1 |
| | VL-S (no. of times) | 2 | 1 | 3 | 2 | × |
| | VL-W (no. of times) | 3 | 2 | 6 | 5 | × |
| Glass plate | SL (minute) YI | 0.5 0.2 | 0.5 0.2 | 1 0.4 | 1 5.5 | 10 0.4 |
| | UV (no. of times) YI | 1 0.2 | 1 0.2 | 1 0.4 | 1 4.8 | 1 0.3 |
| | VL-S (no. of times) YI | 1 0.6 | 1 0.6 | 2 0.4 | 1 5.7 | × |
| | VL-W (no. of times) YI | 1 0.7 | 1 0.8 | 3 0.8 | 2 6.4 | × |

**Table 3**

| | | Ex. 6 | Ex. 7 | Ex. 8 | Ex. 9 | comp. Ex. 6 | comp. Ex. 7 |
|---|---|---|---|---|---|---|---|
| Resin comp. | EA | 75 | 75 | 75 | 75 | 75 | 75 |
| | HEMA | 19 | 19 | 19 | 19 | 19 | 19 |
| | Silane-E | 2 | 2 | 2 | 2 | 2 | 2 |
| Initiator | compound 1-7 | 1 | 1 | 4 | | | |
| | compound 1-9 | | | | 1 | | |
| | DETX-S | | 1 | | | 0.5 | |
| | EDMAB | | | | | 0.5 | |
| | cQ | | | | | 1 | |
| | MAPO | | | | | | 2 |
| Polyca plate | SL (minute) | 1 | 1 | 0.5 | 1 | 1 | 20 |
| | UV (no. of times) | 1 | 1 | 1 | 1 | 1 | 1 |
| | VL-S (no. of times) | 1 | 1 | 1 | 1 | 1 | 1 |
| | VL-W (no. of times) | 3 | 2 | 1 | 4 | 4 | 3 |
| Glass plate | SL (minute) YI | 0.5 0.3 | 0.5 | 0.5 | 0.5 | 1 20.5 | 10 1.2 |
| | UV (no. of times) YI | 1 0.2 | 1 0.4 | 1 0.5 | 1 0.3 | 1 19.9 | 1 0.9 |
| | VL-S (no. of times) YI | 1 0.3 | 1 0.5 | 1 0.6 | 1 0.5 | 1 18.9 | x |
| | VL-W (no. of times) YI | 2 0.4 | 2 0.6 | 1 0.7 | 2 0.5 | 2 19.3 | x |

**Table 4**

| | | Ex. 10 | Ex. 11 | Ex. 12 | Ex. 13 | comp. Ex. 8 |
|---|---|---|---|---|---|---|
| Resin comp. | UA | 35 | 35 | 35 | 35 | 35 |
| | EA | 10 | 10 | 10 | 10 | 10 |
| | A-HD | 15 | 15 | 15 | 15 | 15 |
| | AcMO | 5 | 5 | 5 | 5 | 5 |
| | HEMA | 31 | 31 | 31 | 31 | 31 |
| | Silane-E | 0.5 | 0.5 | 0.5 | 0.5 | 0.5 |
| | Silane-A | 0.5 | 0.5 | 0.5 | 0.5 | 0.5 |
| Initiator | compound 1-2 | 2 | | | | |
| | compound 1-7 | | 2 | 2 | 2 | |
| | DETX-S | | 1 | | 0.5 | 1 |
| | IR184 | 1 | | 1 | 0.5 | |
| | MAPO | | | | | 2 |
| Polyca plate | SL (minute) | 1 | 0.5 | 1 | 0.5 | 5 |
| | UV (no. of times) | 1 | 1 | 1 | 1 | 1 |
| | VL-S (no. of times) | 1 | 1 | 1 | 1 | 2 |
| | VL-W (no. of times) | 1 | 1 | 2 | 1 | 6 |
| Glass plate | SL (minute) YI | 0.5 0.2 | 0.5 2.0 | 0.5 0.3 | 0.5 1.9 | 2 4.3 |
| | UV (no. of times) YI | 1 0.6 | 1 1.2 | 1 0.3 | 1 1.9 | 1 3.2 |
| | VL-S (no. of times) YI | 1 0.6 | 1 1.6 | 1 0.5 | 1 1.2 | 1 3.5 |
| | VL-W (no. of times) YI | 1 0.7 | 1 1.9 | 1 0.6 | 1 1.6 | 3 3.4 |

The values for the resin compositions and the initiators represent parts by weight.

The term "minute" indicates the curing period, the term "no. of times" indicates the number of times the plates were passed through the light source device for UV curing below, and "X" means that curing was insufficient even after the plates were passed through the light source device for UV curing 10 times.

Each resin composition which adhered the two polycarbonate plates and the two glass plates showed such a strong adhesion intensity that the plates could not be detached from each other by hand.

The products obtained by curing the resin compositions obtained in Examples 1 to 13 were little colored and capable of being cured by visible light, compared with those obtained by curing the resin compositions obtained in comparative Examples 1 to 8.

### Example 14

The components shown in Table 5 were mixed to obtain a resin composition.

### Example 15

The components shown in Table 5 were mixed to obtain a resin composition.

### (comparative Example 9)

The components shown in Table 5 were mixed to obtain a resin composition.

### (comparative Example 10)

The components shown in Table 5 were mixed to obtain a resin composition.

### (comparative Example 11)

The components shown in Table 5 were mixed to obtain a resin composition.

### (Test of Inner curing)

The resin compositions obtained in Examples 14 and 15 and comparative Examples 9 to 11 were used for the test of inner curing.

Each of the resin compositions in Table 5 was poured into a round aluminum cup to the depth of ca. 5.5 mm and the cup was passed once through the light source device for UV curing using the lights shown in the above Tables 1 to 4. Then, the film thickness of the cured part (cured from the upper surface) was measured 3 times using slide calipers (at every ca. 60 degrees along the circle line) and the average values were recorded. The evaluation results are shown in Table 5.

**Table 5**

| | | Ex. 14 | Ex. 15 | comp. Ex. 9 | comp. Ex. 10 | comp. Ex. 11 |
|---|---|---|---|---|---|---|
| Resin comp. | APG700 | 80 | 80 | 80 | 80 | 80 |
| | APG200 | 19.8 | 19.8 | 19.8 | 19.8 | 19.8 |
| Initiator | compound 1-2 | 0.2 | | | | |
| | compound 1-7 | | 0.2 | | | |
| | MAPO | | | 0.2 | | |
| | BAPO | | | | 0.2 | |
| | cQ | | | | | 0.2 |
| Film thickness (mm) | UV color of cured product | 5.5 not colored | 5.5 not colored | 5.5 not colored | 5.5 yellow | 5.5 deep yellow |
| | VL-S | 5.5 | 5.5 | 2.8 | 5.3 | 5.0 |
| | VL-W | 5.0 | 5.1 | 0.0 | 0.6 | 0.0 |

The resin compositions obtained in Examples 14 and 15 were excellent in inner curing compared with those obtained in comparative Examples 9 to 11.

### Example 16

The components shown in Table 6 were mixed to obtain a resin composition.

### Example 17

The components shown in Table 6 were mixed to obtain a resin composition.

### (comparative Example 12)

The components shown in Table 6 were mixed to obtain a resin composition.

### (comparative Example 13)

The components shown in Table 6 were mixed to obtain a resin composition.

### (comparative Example 14)

The components shown in Table 6 were mixed to obtain a resin composition.

### (Test of curing of compositions containing Titanium Oxide)

The resin compositions obtained in Examples 16 and 17 and comparative Examples 12 to 14 were used for the test of curing of compositions containing titanium oxide.

Each of the resin compositions in Table 6 and titanium oxide (rutile type) were dispersed using a paint shaker and the resulting composition was poured into an aluminum cup to the depth of ca. 5.5 mm. The cup was passed once through the light source device for UV curing (UV: not shielded). Then, the film thickness of the cured part (cured from the upper surface) was measured 3 times using slide calipers and the average values were recorded. The evaluation results are shown in Table 6.

**Table 6**

| | | Ex. 16 | Ex. 17 | comp. Ex. 12 | comp. Ex. 13 | comp. Ex. 14 |
|---|---|---|---|---|---|---|
| Resin comp. | APG700 | 80 | 80 | 80 | 80 | 80 |
| | APG200 | 19.8 | 19.8 | 19.8 | 19.8 | 19.8 |
| Initiator | compound 1-2 | 0.2 | | | | |
| | compound 1-7 | | 0.2 | | | |
| | MAPO | | | 0.2 | | |
| | BAPO | | | | 0.2 | |
| | cQ | | | | | 0.2 |
| Film thickness (mm) | Titanium oxide content 0 wt% | 5.5 | 5.5 | 5.5 | 5.5 | 5.5 |
| | 0.2 wt% | 5.5 | 5.5 | 2.1 | 2.9 | 2.5 |
| | 0.5 wt% | 3.9 | 4.0 | 1.5 | 2.1 | 1.2 |
| | 1.0 wt% | 2.4 | 2.5 | 1.0 | 1.6 | 0.8 |
| | 1.5 wt% | 2.1 | 2.2 | 0.9 | 1.3 | 0.6 |

The resin compositions obtained in Examples 16 and 17 were excellent in inner curing compared with those obtained in comparative Examples 12 to 14.

### Example 18

The components shown in Table 7 were mixed to obtain a resin composition.

### Example 19

The components shown in Table 7 were mixed to obtain a resin composition.

### (comparative Example 15)

The components shown in Table 7 were mixed to obtain a resin composition.

### (comparative Example 16)

The components shown in Table 7 were mixed to obtain a resin composition.

### (comparative Example 17)

The components shown in Table 7 were mixed to obtain a resin composition.

### (Test of curing of compositions containing a UV Absorber)

The resin compositions obtained in Examples 18 and 19 and comparative Examples 15 to 17 were used for the test of curing of compositions containing a UV absorber.

Each of the resin compositions in Table 7 and UVA (TINUVIN P) were poured into an aluminum cup to the depth of ca. 5.5 mm and the cup was passed once through the light source device for UV curing (UV: not shielded). Then, the film thickness of the cured part (cured from the upper surface) was measured 3 times using slide calipers and the average values were recorded. The evaluation results are shown in Table 7.

**Table 7**

| | | Ex. 18 | Ex. 19 | comp. Ex. 15 | comp. Ex. 16 | comp. Ex. 17 |
|---|---|---|---|---|---|---|
| Resin comp. | APG700 | 80 | 35 | 35 | 35 | 35 |
| | APG200 | 19.8 | 10 | 10 | 10 | 10 |
| Initiator | compound 1-2 | 0.2 | | | | |
| | compound 1-7 | | 0.2 | | | |
| | MAPO | | | 0.2 | | |
| | BAPO | | | | 0.2 | |
| | cQ | | | | | 0.2 |
| Film thickness (mm) | UVA content 0 wt% | 5.5 | 5.5 | 5.5 | 5.5 | 5.5 |
| | 0.2 wt% | 5.5 | 5.5 | 5.4 | 5.5 | 5.1 |
| | 0.7 wt% | 5.5 | 5.5 | 3.2 | 5.5 | 4.7 |
| | 1.0 wt% | 5.5 | 5.5 | 2.9 | 5.4 | 4.5 |
| | 1.5 wt% | 5.4 | 5.4 | 2.4 | 5.3 | 3.4 |
| | 2.0 wt% | 5.4 | 5.4 | 2.0 | 5.3 | 3.1 |

In the table, "wt%" indicates percent by weight.

The resin compositions obtained in Examples 18 and 19 were excellent in inner curing compared with those obtained in comparative Examples 15 to 17.

### Example 20

The components shown in Table 8 below were mixed in a yellow room to obtain a resin composition. When mixed, the components were dissolved by heating to 40ºc, and the resulting mixture was filtered through a membrane filter (0.45 µm, 25N; GL Sciences Inc.) to obtain a resin for nanoimprint. (In the following examples, a resin composition was obtained in the same manner as above.)

### Example 21

The components shown in Table 8 were mixed to obtain a resin for nanoimprint.

### Example 22

The components shown in Table 8 were mixed to obtain a resin for nanoimprint.

### Example 23

The components shown in Table 8 were mixed to obtain a resin for nanoimprint.

### Example 24

The components shown in Table 8 were mixed to obtain a resin for nanoimprint.

### Test Example 1

Several drops of a resin (each of the resins for nanoimprint obtained in Examples 20 to 24) were dropped on a mold of a silicon wafer (diameter: 2 inches) [line convexo-concave type: 200 nm to 2 µm, previously treated with a release agent (Optool DSX; Daikin Industries, Ltd.)], and covered with a polycarbonate plate (thickness: 2 mm, 4 x 4 cm; Nippon Testpanel co., Ltd.), followed by curing by light irradiation at room temperature using a nanoimprint apparatus (Nanoimprinter NM-0401, equipped with a UV irradiator; Meisho Kiko co., Ltd.) (transfer conditions: pressure 50N, 30 seconds; light irradiation, 10 seconds; and release of pressure). Then, the mold was released from the substrate and the transferred pattern was observed using an electron microscope or SEM (scanning electron microscope). The patterns were evaluated from the observation results and the release level.

### Test Example 2

Several drops of a resin (each of the resins for nanoimprint obtained in Examples 20 to 24) were dropped on a mold of a silicon wafer, and covered with a glass plate in the same manner as in Test Example 1. Then, the resin was further covered with a polyimide film (Upilex 50S, 3 x 3 cm; Ube Industries, Ltd.) and cured by light irradiation at room temperature using a nanoimprint apparatus in the same manner as in Test Example 1, followed by evaluation.

### Test Example 3

Several drops of a resin (each of the resins for nanoimprint obtained in Examples 20 to 24) were dropped on a silicon wafer (diameter: 2 inches), and covered with a quartz mold [standard type NIM-PH350; NTT-AT Nanofabrication corporation, previously treated with a release agent (Optool DSX; Daikin Industries, Ltd.)], followed by curing by light irradiation from the side of the quartz mold at room temperature using a nanoimprint apparatus (Nanoimprinter NM-0401, equipped with a UV irradiator; Meisho Kiko co., Ltd.) (transfer conditions: pressure 50N, 30 seconds; light irradiation, 10 seconds; and release of pressure). Then, the mold was released from the substrate and the transferred pattern was observed using an electron microscope or SEM (scanning electron microscope). The patterns were evaluated from the observation results and the release level.

The results of Test Examples 1 to 3 are shown in Table 8.

**Table 8**

| | components or substrate | Ex. 20 | Ex. 21 | Ex. 22 | Ex. 23 | Ex. 24 |
|---|---|---|---|---|---|---|
| Initiator | compound 1-7 | 1 | 1 | 1 | 1 | |
| | compound 1-8 | | | | | 1 |
| Resin comp. | TPGDA | 60 | 30 | | 30 | 60 |
| | NVP | 29 | 29 | 29 | 29 | 29 |
| | TMPTA | 10 | 10 | 10 | 10 | 10 |
| | ADcP | | 30 | | | |
| | A200 | | | 60 | | |
| | APG700 | | | | 30 | |
| Test Ex. 1 | Polycarbonate | o | o | o | o | o |
| Test Ex. 2 | Glass plate + polyimide | o | o | o | o | o |
| Test Ex. 3 | Silicon | o | o | o | o | o |

In Table 8, the values for the resin compositions and the initiators indicate parts by weight. In the examples in Table 8, o indicates good transfer and release level, and x indicates incomplete transfer.

### (Nanoimprint Test)

The abbreviations used in Table 8 are explained below.
TPGDA: tripropylene glycol diacrylate (NK Ester APG-200; Shin-Nakamura chemical co., Ltd.)
NVP: N-vinyl pyrrolidone (Tokyo chemical Industry co., Ltd.)
TMPTA: trimethylolpropane triacrylate (NK Ester A-TMPT; Shin-Nakamura chemical co., Ltd.)
A200: polyethylene glycol diacrylate #200 (NK Ester A-200; Shin-Nakamura chemical co., Ltd.)
ADcP: tricyclodecanedimethanol diacrylate (NK Ester A-DcP; Shin-Nakamura chemical co., Ltd.)
APG700: polypropylene glycol diacrylate #700 (NK Ester APG-700; Shin-Nakamura chemical co., Ltd.)

As can be seen from Table 8, the resins for nanoimprint obtained in Examples 20 to 24 are capable of good pattern transfer and excellent in release properties.

### Industrial Applicability

The present invention can provide a resin composition which can be cured by visible light and whose cured product is not colored or little colored, and the like.

## Claims

1. A resin composition comprising a phosphine oxide compound represented by general formula (1): 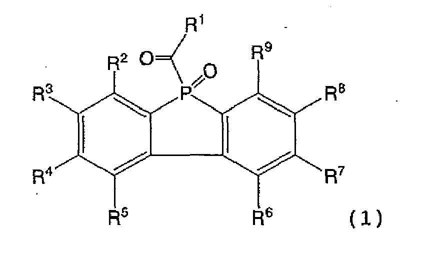 (wherein R¹ represents alkyl, alkenyl, alkynyl, cycloalkyl, cycloalkenyl, substituted or unsubstituted aryl, substituted or unsubstituted heteroaryl, substituted or unsubstituted aralkyl, or substituted or unsubstituted aralkenyl; and R², R³, R⁴, R⁵, R⁶, R⁷, R⁸ and R⁹, which may be the same or different, each represent a hydrogen atom, a halogen atom, alkyl, alkenyl, alkynyl, cycloalkenyl, alkoxy, cycloalkyloxy, alkenyloxy, cycloalkenyloxy, alkynyloxy, aryloxy, heteroaryloxy, aralkyloxy, substituted or unsubstituted aryl, substituted or unsubstituted heteroaryl, substituted or unsubstituted aralkyl, or substituted or unsubstituted aralkenyl, and two adjacent groups among R², R³, R⁴, R⁵, R⁶, R⁷, R⁸ and R⁹ may form a hydrocarbon ring together with the two carbon atoms adjacent thereto) and an unsaturated compound.

2. The resin composition according to claim 1, which comprises the phosphine oxide compound wherein R¹ is substituted or unsubstituted aryl or substituted or unsubstituted heteroaryl, and R², R³, R⁴, R⁵, R⁶, R⁷, R⁸ and R⁹ each are a hydrogen atom.

3. The resin composition according to claim 1, which comprises the phosphine oxide compound wherein R¹ is substituted or unsubstituted aryl, and R^{2,} R^{3,} R^{4,} R⁵, R⁶, R⁷, R⁸ and R⁹ each are a hydrogen atom.

4. An adhesive comprising the resin composition according to any of claims 1 to 3.

5. A cured product obtained by curing the resin composition according to any of claims 1 to 3 by light irradiation.

6. A resin for nanoimprint comprising the resin composition according to any of claims 1 to 3.
